# EUROPEAN PATENT APPLICATION

(11) **EP 4 245 772 A1**
(43) Date of publication of application: **20.09.2023**
(21) Application number: 22305325.7
(22) Date of filing: 18.03.2022
(51) Int. Cl.: C07K 16/22, A61P 1/16, A61K 39/00

(54) **ANTI-NETRIN-1 ANTIBODY TO TREAT LIVER INFLAMMATION**

(71) Applicant: Netris Pharma, 69008 Lyon (FR)
(72) Inventor: BARNAULT, Romain, 69364 LYON CEDEX 07 (FR); VERZEROLI, Claire, 69424 LYON CEDEX 03 (FR); FOURNIER, Carole, 38700 LA TRONCHE (FR); MEHLEN, Patrick, 69008 LYON (FR); GIBERT, Benjamin, 69008 LYON (FR); ZOULIM, Fabien, 69424 LYON CEDEX 03 (FR); PARENT, Romain, 69424 LYON CEDEX 03 (FR)
(74) Representative: Lavoix

(57) **Abstract**

The netrin-1 protein displays anti-inflammatory activity in the liver and is an unexpected proinflammatory factor in CLD. The present invention provides for blocking or capturing netrin-1 in the liver for treating liver inflammation, and thus methods for treating liver inflammation. To perform this, the invention makes use of a polyclonal or monoclonal antibody specifically binding to netrin-1, including a series of humanized monoclonal antibodies.

## Description

The present invention concerns blocking or capturing netrin-1 in the liver for treating liver (or hepatic) inflammation, and thus methods for treating liver inflammation.

### Background of invention

Chronic liver diseases (CLDs) affect more than 1 billion people worldwide. They represent the main etiological conditions for the onset of liver injury, impaired liver function (cirrhosis), and hepatocellular carcinoma (HCC). Though CLDs exhibit a high degree of diversity in terms of causal effects, including infectious (hepatitis B virus HBV and hepatitis C viruses HCV), metabolic, toxic, and genetic, they all converge towards hepatic inflammation. Inflammation in turn drives hepatocyte turnover, extracellular matrix accumulation, histological worsening and the long-term induction of tumorigenic mediators such as IL-6, eventually leading to the development of HCC. A clear understanding of the onset of this inflammatory response and of its outcome in the hepatic tissue is hampered by biologically-opposite functions, as it favours viral and dead cell clearance, as well as histological wound healing, but exacerbates fibrogenesis, thus inducing cirrhosis and oncogenesis.

Netrin-1 is well-known for preventing tumoral cells apoptosis through its binding to the so-called dependence receptors (3-5), and has been demonstrated that it is HBV-HCV induced (6) and endowed hepatocytes with resistance to apoptosis during the unfolded protein response (UPR) (7), a hallmark of CLDs and cirrhosis.

### Summary of invention

Netrin-1 is identified as a hepatic inflammation-inducible factor. During exploration of the impact of a phase 2 clinical trial-tested humanized anti-netrin-1 antibody (NP137) in two distinct, TLR2/TLR3/TLR6-dependent, hepatic inflammatory mouse settings, a clear and unexpected anti-inflammatory activity of netrin-1 has been observed. In particular, the proinflammatory impact of netrin-1 on several chemokines and Ly6C+ macrophages has been detected. The experiments presented hereinafter identify netrin-1 not only as an inflammation-inducible factor in the liver, but also as a contributor to hepatic inflammation. Especially, the humanized anti-netrin capture antibody NP137 (23) (US Clinical Trials #02977195) displays anti-inflammatory activity in the liver and identifies netrin-1 as a previously unknown and unexpected proinflammatory factor in CLD.

A first object of the invention is thus an anti-netrin-1 antibody for use as an anti-inflammatory medicament, especially in the treatment of liver inflammation.

Another object of the invention is thus an anti-netrin-1 antibody for use in the treatment of liver inflammation.

Another object of the invention is the use of an anti-netrin-1 antibody for the manufacture of a medicament for the treatment of liver inflammation.

Another object of the invention is a method for treating inflammation, especially liver inflammation in patient in need thereof, comprising administering said patient with an efficient amount of an anti-netrin-1 antibody.

The humanized anti-netrin (capture) antibody NP137 displays anti-inflammatory activity in the liver and identifies netrin-1 as a previously unknown proinflammatory factor in CLD. In an aspect, by binding to netrin-1, the anti-netrin-1 antibodies of the invention inhibit the proinflammatory function of netrin-1 in the liver. In particular, the anti-netrin-1 antibody blocks or captures netrin-1 protein in the liver. Thus, the use and methods of use of the invention are deemed inhibiting the liver inflammation due to, or involving, netrin-1 presence and/or activity in the liver. In an aspect, the liver inflammation is one wherein netrin-1 is present and has a proinflammatory action in the liver. In an aspect, by blocking or capturing netrin-1, the antibody inhibits the proinflammatory impact of netrin-1 on several chemokines and Ly6C+ macrophages, and the antibody reveals a clear anti-inflammatory activity. In particular, it is deemed herein the antibody is reversing the induction of one or several transcripts, such as the chemokines *Ccl2*, *Ccl3, Ccl5, Ccl8,* and/or one or several actors related to the inflammation-associated interferon system, such as *Stat1, Oas1a, Oasl1, Ifi44*, and/or of the master regulator of inflammation cyclooxygenase *Ptgs2* and its product prostaglandin I2 (*Ptgir*)*.* It is also deemed the antibody is reversing the transcripts encoding the chemoattractant *Cxcl10, Ccl2* and *Ccl5,* the canonical inflammatory cytokine *IL1B*, the macrophage inflammatory protein *Mip2* and the *Stat1* and *Gnb1* transducers.

The liver may be a healthy liver with a risk of inflammation appearance, or an inflammatory liver, and preferably with no cancerous status.

The presence of hepatic inflammation in a patient may be detected by the methods in use in the clinic and in accordance with clinical or biopsy observations, measurements of marker values or thresholds, defining liver inflammation. Determination may be made on liver biopsy (e.g. by Western blot using a known scale, semi-quantitative) or, more conveniently, on blood samples. Hepatic inflammation may be assessed, by measuring aminotransferase (especially alanine or aspartate aminotransferase) levels in the blood or serum, and high levels known by the clinician are characteristic of liver/hepatic inflammation. In particular, liver specific alanine aminotransferase (ALT) level is measured, and a high level denotes hepatic inflammation, and/or the C-Reactive protein (CRP) level is measured and a high level known by the clinician denotes liver/hepatic inflammation. One may also use the hepatic activity index or score on liver histology analysis, and any other available method. It is also possible to use transcripts of the inflammatory cytokines mentioned in the examples, and their corresponding proteins. Intrahepatic leucocytes (immune infiltrate) and macrophages are also good markers.

The therapeutic indication may be a liver inflammation (or hepatic inflammation) or a liver inflammation associated with a disease such as : a liver infection (e.g. hepatitis B virus, hepatitis C virus); a metabolic disease; a drug induced liver injury (DILI) ; a chronic liver disease (CLD) whatever its etiology (e.g. viral infection, metabolic disease, alcohol related liver disease, autoimmune liver disease etc.); a toxic liver injury; an impaired liver function; cirrhosis; a genetic liver disease.

The antibody may be a polyclonal or monoclonal antibody specifically binding to netrin-1 (NTN1) (anti-netrin-1 antibody or antibody binding to netrin-1), especially human netrin-1.

An anti-netrin-1 polyclonal antibody may, inter alia, be obtained by immunizing an animal such as a rabbit, a mouse and the like with the aid of the selected amino acid sequence, collecting and then depleting the antiserum obtained on, for example, an immunoadsorbent containing the receptor according to methods known per se to a person skilled in the art.

The netrin-1 amino acid sequence is as depicted on SEQ ID NO: 1 and netrin-1 may be used in whole or in part to generate polyclonal or monoclonal antibodies.

Generally, monoclonal antibodies may be obtained according to the conventional method of lymphocyte fusion and hybridoma culture described by Köhler and Milstein, (Nature, 1975, 256(5517): 495-7). Other methods for preparing monoclonal antibodies are also known (Harlow et al., ed., 1988 "Antibodies: a laboratory manual"). The monoclonal antibodies may be prepared by immunizing a mammal (for example a mouse, a rat, a rabbit or even a human being, and the like) and using the lymphocyte fusion technique leading to hybridoma (Köhler and Milstein, 1975). Alternative techniques to this customary technique exist. It is possible, for example, to produce monoclonal antibodies by expressing a nucleic acid cloned from a hybridoma. It is also possible to produce antibodies by the phage display technique by introducing cDNAs for antibodies into vectors, which are typically filamentous phages which exhibit gene libraries V at the surface of the phage (for example fUSE5 for E. *coli,* Scott J.K., Smith G.P. Science 1990; 249:386-390). Protocols for constructing these antibody libraries are described in J.D. Marks et al., J. Mol. Biol., 222 (1991), p. 581). The cDNA corresponding to full length netrin-1 with signal sequence (SEQ ID NO: 2) or to a suitable fragment thereof may be used to produce monoclonal antibodies according to these methods.

The anti-netrin-1 monoclonal antibody (mAb) may be a murine, a chimeric, a humanized or a full-human monoclonal antibody. The fragment may be any type of mAb fragment that keeps substantially the ability of the whole antibody to bind to Netrin-1, it can be for example a Fab or a F(ab')₂. In particular, a monoclonal antibody is one disclosed in WO2015/104360 or US10,494,427, which documents are incorporated herein by reference, and disclose useful murine, chimeric and humanized monoclonal antibodies. These are antibodies or fragments thereof, which specifically bind to a NTN1 epitope or polypeptide having the amino acid sequence SEQ ID NO: 3 or 33, or a variant thereof.

The antibodies useful in the invention may be defined by their CDRs. In particular these CDRs are derived from the murine antibody 4C11 disclosed in WO2015/104360, which specifically binds to the polypeptide having the amino acid sequence SEQ ID NO: 3 or 33. Preferably, the antibody is a monoclonal antibody or an antigen-binding fragment thereof, comprising:
a variable domain VH comprising:
   - a H-CDR1 having a sequence set forth as SEQ ID NO: 5;
   - a H-CDR2 having a sequence set forth as SEQ ID NO: 6;
   - a H-CDR3 having a sequence set forth as SEQ ID NO: 7;
a variable domain VL comprising:
   - a L-CDR1 having a sequence set forth as SEQ ID NO: 8;
   - a L-CDR2 having a sequence YAS;
   - a L-CDR3 having a sequence set forth as SEQ ID NO: 9;
      or
a variable domain VH comprising:
   - a H-CDR1 having a sequence set forth as SEQ ID NO: 28;
   - a H-CDR2 having a sequence set forth as SEQ ID NO: 29;
   - a H-CDR3 having a sequence set forth as SEQ ID NO: 30;
a variable domain VL comprising:
   - a L-CDR1 having a sequence set forth as SEQ ID NO: 31;
   - a L-CDR2 having a sequence set forth as SEQ ID NO: 32;
   - a L-CDR3 having a sequence set forth as SEQ ID NO: 9.

In a first series of embodiments, the antibody of the invention comprises an amino acid sequence SEQ ID NO: 10, 11, 12 or 13. Typically, it comprises a VH of sequence SEQ ID NO: 10 and a VL of sequence SEQ ID NO: 11, or a heavy chain of sequence SEQ ID NO: 12 and a light chain of sequence SEQ ID NO: 13.

In a second series of embodiments, the antibody is chimeric. Preferably, it comprises a VH of sequence SEQ ID NO: 27 and a VL of sequence SEQ ID NO: 19.

In a third series of embodiments, the antibody is humanized. Preferably, it comprises an amino acid sequence selected from the group of SEQ ID NO: 14 to 18 (VL) and/or from the group of SEQ ID NO: 20 to 26 (VH). Typically, the antibody is humanized and comprises a VH having an amino acid sequence selected from the group of SEQ ID NO: 14 to 18 and a VL having an amino acid sequence selected from the group of SEQ ID NO: 20 to 26.

Specific embodiments disclosed in this prior document and that can be used herein are the following antibodies listed in Table 1. The first listed antibody is a chimeric 4C11 antibody, comprising the murine VH and VL of the murine 4C11 antibody. HUM00 listed in Table 1 corresponds to the grafting of the murine 4C11 CDRs into a human IgG1. The ten humanized mAb HUM01 to HUM10 correspond to humanized mAbs derived from HUM00 with the same CDRs, but specific modifications in the FR regions of the human IgG. HUM03 is also publicly known as NP137 and is currently under clinical trials. Sequences of the human IgG1 CH come from Genbank AEL33691.1 modified R97K. Sequences of the human IgG1 CL (Kappa) come from Genbank CAC20459.1. The other allotypes may be used as well. Specific binding of all these mAbs, murine, chimeric and humanized HUM01-HUM10, Fab fragments and F(ab')₂ fragments, to Netrin-1 and their ability to inhibit binding of netrin-1 to its receptor UNC5B, are demonstrated in US 10,494,427 (Example 3).

In particular, these antibodies specifically bind to the polypeptide having the amino acid sequence SEQ ID NO: 33.

| Table 1 | VH SEQ ID NO: | Constant heavy chain | VL SEQ ID NO: | Constant light chain |
|---|---|---|---|---|
| 4C11 | 10 | Human IgG1 | 11 | Human IgG1 |
| HUM00 | 27 | Human IgG1 | 19 | Human IgG1 |
| HUM01 | 20 | Human IgG1 | 14 | Human IgG1 |
| HUM02 | 21 | Human IgG1 | 15 | Human IgG1 |
| HUM03 | 22 | Human IgG1 | 16 | Human IgG1 |
| HUM04 | 23 | Human IgG1 | 17 | Human IgG1 |
| HUM05 | 24 | Human IgG1 | 17 | Human IgG1 |
| HUM06 | 25 | Human IgG1 | 16 | Human IgG1 |
| HUM07 | 26 | Human IgG1 | 17 | Human IgG1 |
| HUM08 | 22 | Human IgG1 | 17 | Human IgG1 |
| HUM09 | 25 | Human IgG1 | 18 | Human IgG1 |
| HUM10 | 21 | Human IgG1 | 16 | Human IgG1 |

Preferably, the antibody is a monoclonal antibody or an antigen-binding fragment thereof, comprising a pair of VH and VL sequences selected from the following pairs: SEQ ID NO: 27 and 19, SEQ ID NO: 20 and 14, SEQ ID NO: 21 and 15, SEQ ID NO: 22 and 16, SEQ ID NO: 23 and 17, SEQ ID NO: 24 and 17, SEQ ID NO: 25 and 16, SEQ ID NO: 26 and 17, SEQ ID NO: 22 and 17, SEQ ID NO: 25 and 18, SEQ ID NO: 21 and 16. More preferably, the antibody is a monoclonal antibody or an antigen-binding fragment thereof, comprising a pair of VH and VL sequences SEQ ID NO: 22 and 16.

The anti-netrin-1 antibody may further comprise a Human IgG1 Constant heavy chain (CH) and/or a Human IgG1 Constant light chain (CL), in particular a human kappa constant domain.

In an embodiment, sequences of the human IgG1 CH come from Genbank AEL33691.1 modified R97K. Sequences of the human IgG1 CL (Kappa) come from Genbank CAC20459.1. In an embodiment, the mAb is NP137 (AB_2811180 in The Antibody Registry) and comprises SEQ ID NO: 22 and 16 as VH, respectively VL sequences, and those specific IgG1 CH and CL.

As anti-netrin-1 antibodies that may be used, one may cite other antibodies, especially monoclonal antibodies, or their antigen-binding fragments, developed against human netrin-1 or against animal netrin-1, netrin-1 being very homologous among species. May be cited: Abcam antibodies ab126729, ab122903, ab201324, ab39370; AF1109, AF6419, AF128.

**Table 2: Description of the sequences:**

| SEQ ID NO: | Description | Sequence |
|---|---|---|
| 1 | NTN1 amino acid (aa) sequence (seq.) with signal peptide in bold and linear epitope mapping in bold and underlined | MMRAVWEALAALAAVACLVGAVRGGPGLSMFAGQAA QPDPCSDENGHPRRCIPDFVNAAFGKDVRVSSTCGRP PARYCVVSERGEERLRSCHLCNASDPKKAHPPAFLTDL NNPHNLTCWQSENYLQFPHNVTLTLSLGKKFEVTYVSL QFCSPRPESMAIYKSMDYGRTWVPFQFYSTQCRKMYN RPHRAPITKQNEQEAVCTDSHTDMRPLSGGLIAFSTLDG RPSAHDFDNSPVLQDWVTATDIRVAFSRLHTFGDENED DSELARDSYFYAVSDLQVGGRCKCNGHAARCVRDRDD SLVCDCRHNTAGPECDRCKPFHYDRPWQRATAREANE CVACNCNLHARRCRFNMELYKLSGRKSGGVCLNCRH NTAGRHCHYCKEGYYRDMGKPITHRKACKACDCHPVG AAGKTCNQTTGQCPCKDGVTGITCNRCAKGYQQSRSPI |
| | | APCIKIPVAPPTTAASSVEEPEDCDSYCKASKGLKINMK KYCKKDYAVQIHILKADKAGDWWKFTVNIISVYKQGTSRI RRGDQSLWIRSRDIACKCPKIKPLKKYLLLGNAEDSPDQ SGIVADKSSLVIQWRDTWARRLRKFQQREKKGKCKKA |
| 2 | NTN1 nucleic acid seq. | ATGATGCGCGCAGTGTGGGAGGCGCTGGCGGCGCT GGCGGCGGTGGCGTGCCTGGTGGGCGCGGTGCGCG GCGGGCCCGGGCTCAGCATGTTCGCGGGCCAGGCG GCGCAGCCCGATCCCTGCTCGGACGAGAACGGCCAC CCGCGCCGCTGCATCCCGGACTTTGTCAATGCGGCC TTCGGCAAGGACGTGCGCGTGTCCAGCACCTGCGGC CGGCCCCCGGCGCGCTACTGCGTGGTGAGCGAGCG CGGCGAGGAGCGGCTGCGCTCGTGCCACCTCTGCAA CGCGTCCGACCCCAAGAAGGCGCACCCGCCCGCCTT CCTCACCGACCTCAACAACCCGCACAACCTGACGTG CTGGCAGTCCGAGAACTACCTGCAGTTCCCGCACAA CGTCACGCTCACACTGTCCCTCGGCAAGAAGTTCGAA GTGACCTACGTGAGCCTGCAGTTCTGCTCGCCGCGG CCCGAGTCCATGGCCATCTACAAGTCCATGGACTACG GGCGCACGTGGGTGCCCTTCCAGTTCTACTCCACGC AGTGCCGCAAGATGTACAACCGGCCGCACCGCGCGC CCATCACCAAGCAGAACGAGCAGGAGGCCGTGTGCA CCGACTCGCACACCGACATGCGCCCGCTCTCGGGCG GCCTCATCGCCTTCAGCACGCTGGACGGGCGGCCCT CGGCGCACGACTTCGACAACTCGCCCGTGCTGCAGG ACTGGGTCACGGCCACAGACATCCGCGTGGCCTTCA GCCGCCTGCACACGTTCGGCGACGAGAACGAGGAC GACTCGGAGCTGGCGCGCGACTCGTACTTCTACGCG GTGTCCGACCTGCAGGTGGGCGGCCGGTGCAAGTG CAACGGCCACGCGGCCCGCTGCGTGCGCGACCGCG ACGACAGCCTGGTGTGCGACTGCAGGCACAACACGG CCGGCCCGGAGTGCGACCGCTGCAAGCCCTTCCACT ACGACCGGCCCTGGCAGCGCGCCACAGCCCGCGAA GCCAACGAGTGCGTGGCCTGTAACTGCAACCTGCAT GCCCGGCGCTGCCGCTTCAACATGGAGCTCTACAAG CTTTCGGGGCGCAAGAGCGGAGGTGTCTGCCTCAAC TGTCGCCACAACACCGCCGGCCGCCACTGCCATTAC |
| | | TGCAAGGAGGGCTACTACCGCGACATGGGCAAGCCC ATCACCCACCGGAAGGCCTGCAAAGCCTGTGATTGC CACCCTGTGGGTGCTGCTGGCAAAACCTGCAACCAA ACCACCGGCCAGTGTCCCTGCAAGGACGGCGTGACG GGTATCACCTGCAACCGCTGCGCCAAAGGCTACCAG CAGAGCCGCTCTCCCATCGCCCCCTGCATAAAGATC CCTGTAGCGCCGCCGACGACTGCAGCCAGCAGCGTG GAGGAGCCTGAAGACTGCGATTCCTACTGCAAGGCC TCCAAGGGGAAGCTGAAGATTAACATGAAAAAGTACT GCAAGAAGGACTATGCCGTCCAGATCCACATCCTGAA GGCGGACAAGGCGGGGGACTGGTGGAAGTTCACGG TGAACATCATCTCCGTGTATAAGCAGGGCACGAGCC GCATCCGCCGCGGTGACCAGAGCCTGTGGATCCGCT CGCGGGACATCGCCTGCAAGTGTCCCAAAATCAAGC CCCTCAAGAAGTACCTGCTGCTGGGCAACGCGGAGG ACTCTCCGGACCAGAGCGGCATCGTGGCCGATAAAA GCAGCCTGGTGATCCAGTGGCGGGACACGTGGGCG CGGCGGCTGCGCAAGTTCCAGCAGCGTGAGAAGAAG GGCAAGTGCAAGAAGGCCTAGCG |
| 3 | NTN1 aa epitopic seq. | VACNCNLHARRCRFNMELYKLSGRKSGGVCLNCRHNT AGRHCH |
| 4 | NTN1 epitopic cDNA seq. | GTGGCCTGTAACTGCAACCTGCATGCCCGGCGCTGC CGCTTCAACATGGAGCTCTACAAGCTTTCGGGGCGC AAGAGCGGAGGTGTCTGCCTCAACTGTCGCCACAAC ACCGCCGGCCGCCACTGCCAT |
| 5 | aa seq. of CDR1-H (IMGT) | GYTFTSYN |
| 6 | aa seq. of CDR2-H (IMGT) | IYPGNGDT |
| 7 | aa seq. of CDR3-H (IMGT) | ARGGTGFAY |
| 8 | aa seq. of CDR1-L (IMGT) | QSVSND |
| - | aa seq. of CDR2-L (IMGT) | YAS |
| 9 | aa seq. of CDR3-L (IMGT et Kabat) | QQDYSSPWT |
| 10 | aa sequence of mouse 4C11 VH | QAYLQQSGAELVRPGASVKMSCKASGYTFTSYNMHWV KQTPRQGLEWIGAIYPGNGDTSYNQKFKGKATLTVDKS SSTAYMQLSSLTSEDSAVYFCARGGTGFAYWGQGTLV TVSA |
| 11 | aa sequence of mouse 4C11 VL | SIVMTQTPKFLLVSAGDRVTITCKASQSVSNDVAWYQQ KPGQSPKLLIYYASNRYTGVPDRFTGSGYGTDFTFTIST VQAEDLAVYFCQQDYSSPWTFGGGTKLEIK |
| 12 | Full aa sequence of 4C11 (VH + mouse IgG1 CH) | QAYLQQSGAELVRPGASVKMSCKASGYTFTSYNMHWV KQTPRQGLEWIGAIYPGNGDTSYNQKFKGKATLTVDKS SSTAYMQLSSLTSEDSAVYFCARGGTGFAYWGQGTLV TVSAAKTTPPSVYPLAPGSAAQTNSMVTLGCLVKGYFP EPVTVTWNSGSLSSGVHTFPAVLESDLYTLSSSVTVPSS PRPSETVTCNVAHPASSTKVDKKIVPRDCGCKPCICTVP EVSSVFIFPPKPKDVLTITLTPKVTCVVVDISKDDPEVQF SWFVDDVEVHTAQTQPREEQFNSTFRSVSELPIMHQD WLNGKEFKCRVNSAAFPAPIEKTISKTKGRPKAPQVYTI PPPKEQMAKDKVSLTCMITDFFPEDITVEWQWNGQPAE NYKNTQPIMNTNGSYFVYSKLNVQKSNWEAGNTFTCSV LHEGLHNHHTEKSLSHSPGK |
| 13 | Full aa sequence of 4C11 (VL + mouse Kappa CL) | SIVMTQTPKFLLVSAGDRVTITCKASQSVSNDVAWYQQ KPGQSPKLLIYYASNRYTGVPDRFTGSGYGTDFTFTIST VQAEDLAVYFCQQDYSSPWTFGGGTKLEIKRADAAPTV SIFPPSSEQLTSGGASVVCFLNNFYPKDINVKWKIDGSE RQNGVLNSWTDQDSKDSTYSMSSTLTLTKDEYERHNS YTCEATHKTSTSPIVKSFNRNEC |
| 14 | VL aa sequence of humanized variant of 4C11 | EIVMTQSPATLSVSPGERATLSCKASQSVSNDVAWYQQ KPGKAPKLLIYYASNRYTGIPPRFSGSGYGTDFTLTINNI ESEDAAYYFCQQDYSSPWTFGQG |
| 15 | VL aa sequence of humanized variant of | DIQMTQSPSSLSASVGDRVTITCKASQSVSNDVAWFQQ RPGQSPRRLIYYASNRYTGVPSRFSGSGSGTDFTFTISS |
| | 4C11 | LEAEDAATYYCQQDYSSPWTFGQG |
| 16 | VL aa sequence of humanized variant of 4C11 | DIQMTQSPSSLSASVGDRVTITCKASQSVSNDVAWYQQ KPGQAPRLLIYYASNRYTGIPPRFSGSGYGTDFTLTINNI ESEDAAYYFCQQDYSSPWTFGQG |
| 17 | VL aa sequence of humanized variant of 4C11 | DIQMTQSPSSLSASVGDRVTITCKASQSVSNDVAWYLQ KPGQSPQLLIYYASNRYTGVPSRFSGSGSGTDFTFTISS LEAEDAATYYCQQDYSSPWTFGQG |
| 18 | VL aa sequence of humanized variant of 4C11 | DIVMTQTPLSLPVTPGEPASISCKASQSVSNDVAWYQQ KPGQAPRLLIYYASNRYTGIPPRFSGSGYGTDFTLTINNI ESEDAAYYFCQQDYSSPWTFGQG |
| 19 | VL aa sequence of humanized variant of 4C11 | EIVMTQSPATLSVSPGERATLSCRASQSVSNDVAWYQQ KPGQAPRLLIYYASNRYTGIPARFSGSGSGTEFTLTISSL QSEDFAVYYCQQDYSSPWTFGQG |
| 20 | VH aa sequence of humanized variant of 4C11 | QVQLVQSGAEVKKPGASVKVSCKASGYTFTSYNMHWV RQATGQGLEWMGAIYPGNGDTSYNQKFKGRVTITADK STSTAYMELSSLRSEDTAVYYCARGGTGFAYWGQG |
| 21 | VH aa sequence of humanized variant of 4C11 | QVQLQQSGPGLVKPSQTLSLTCAISGYTFTSYNMHWIR QPPGKGLEWIGAIYPGNGDTSYNQKFKGRVTITADKSTS TAYMELSSLRSEDTAVYYCARGGTGFAYWGQG |
| 22 | VH aa sequence of humanized variant of 4C11 | QVQLQQSGPGLVKPSQTLSLTCAISGYTFTSYNMHWVR QATGQGLEWMGAIYPGNGDTSYNQKFKGRLTISKDTSK NQVVLTMTNMDPVDTATYYCARGGTGFAYWGQG |
| 23 | VH aa sequence of humanized variant of 4C11 | EVQLVQSGAEVKKPGESLRISCKGSGYTFTSYNMHWV RQATGQGLEWMGAIYPGNGDTSYNQKFKGRFTISRDD SKNTAYLQMNSLKTEDTAVYYCARGGTGFAYWGQG |
| 24 | VH aa sequence of humanized variant of 4C11 | QVQLQESGPGLVKPSQTLSLTCTVSGYTFTSYNMHWV RQAPGQGLEWMGAIYPGNGDTSYNQKFKGRVTISVDT SKNQFSLKLSSVTAADTAVYYCARGGTGFAYWGQG |
| 25 | VH aa sequence of humanized variant of 4C11 | QVQLVQSGAEVKKPGASVKVSCKASGYTFTSYNMHWV RQATGQGLEWMGAIYPGNGDTSYNQKFKGRVTITADK STSTAYMELSSLRSEDTAVYYCARGGTGFAYWGQG |
| 26 | VH aa sequence of humanized variant of 4C11 | QVQLQQSGPGLVKPSQTLSLTCAISGYTFTSYNMHWVR QATGQGLEWMGAIYPGNGDTSYNQKFKGRVTITADKST STAYMELSSLRSEDTAVYYCARGGTGFAYWGQG |
| 27 | VH aa sequence of | QVQLVQSGAEVKKPGASVKVSCKASGYTFTSYNMHWV |
| | humanized variant of 4C11 | RQAPGQGLEWMGAIYPGNGDTSYAQKFKGRVTMTRDT STSTVYMELSSLRSEDTAVYYCARGGTGFAYWGQ |
| 28 | aa seq. of CDR1-H (Kabat) | SYNMH |
| 29 | aa seq. of CDR2-H (Kabat) | AIYPGNGDTSYNQKFKG |
| 30 | aa seq. of CDR3-H (Kabat) | GGTGFAY |
| 31 | aa seq. of CDR1-L (Kabat) | KASQSVSNDVA |
| 32 | aa seq. of CDR2-L (Kabat) | YASNRYT |
| 33 | NTN1 aa epitopic seq. | ARRCRFNMELYKLSGRKSGGVC |
| 34 | NTN1 epitopic cDNA seq. | GCCCGGCGCTGCCGCTTCAACATGGAGCTCTACAAG CTTTCGGGGCGCAAGAGCGGAGGTGTCTGC |

| | | |
|---|---|---|
| CDRs under IMGT are highlighted in bold in Table 1 where appropriate. | | |

### Definitions and further embodiments, variants and alternatives of the invention:

As used herein, a sequence "at least 85% identical to a reference sequence" is a sequence having, on its entire length, 85%, or more, in particular 90%, 91%, 92%, 93%, 94%, 95%, 96%, 97%, 98%, 99%, 99.5%, 99.6%, 99.7%, 99.8%, 99.9% or 100% sequence identity with the entire length of the reference sequence.

A percentage of "sequence identity" may be determined by comparing the two sequences, optimally aligned over a comparison window, wherein the portion of the polypeptide sequence in the comparison window may comprise additions or deletions (*i.e.* gaps) as compared to the reference sequence (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison is conducted by global pairwise alignment, e.g. using the algorithm of Needleman and Wunsch (1970) J. Mol. Biol. 48: 443. The percentage of sequence identity can be readily determined for instance using the program Needle, with the BLOSUM62 matrix, and the following parameters gap-open=10, gap-extend=0.5.

In the context of the invention, a "conservative amino acid substitution" is one in which an amino acid residue is substituted by another amino acid residue having a side chain group with similar chemical properties (e.g., charge or hydrophobicity). In general, a conservative amino acid substitution will not substantially change the functional properties of a protein. Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Conservative amino acids substitution groups are: valine-leucine-isoleucine, phenylalanine-tyrosine-tryptophan, lysine-arginine, alanine-valine, glutamate-aspartate, and asparagine-glutamine.

Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (*i.e.* "consisting of").

An "antibody" may be a natural or conventional antibody in which two heavy chains are linked to each other by disulfide bonds and each heavy chain is linked to a light chain by a disulfide bond. There are two types of light chain, lambda (λ) and kappa (K). There are five main heavy chain classes (or isotypes) which determine the functional activity of an antibody molecule: IgM, IgD, IgG, IgA and IgE. Each chain contains distinct sequence domains. The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site.

"Complementarity Determining Regions" or "CDRs" refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region.

"Framework Regions" (FRs) refer to amino acid sequences interposed between CDRs, *i.e.* to those portions of immunoglobulin light and heavy chain variable regions that are relatively conserved among different immunoglobulins in a single species. The light and heavy chains of an immunoglobulin each have four FRs, designated FR1-L, FR2-L, FR3-L, FR4-L, and FR1-H, FR2-H, FR3-H, FR4-H, respectively.

As used herein, a "human framework region" is a framework region that is substantially identical (about 85%, or more, in particular 90%, 95%, 97%, 99% or 100%) to the framework region of a naturally occurring human antibody.

In the context of the invention, CDR/FR definition in an immunoglobulin light or heavy chain is to be determined based on Kabat or IMGT definitions.

The residues in antibody variable domains are conventionally numbered according to a system devised by Kabat et al. This system is set forth in Kabat et al., 1987, in Sequences of Proteins of Immunological Interest, US Department of Health and Human Services, NIH, USA (hereafter "Kabat et al."). This numbering system is used in the present specification. The Kabat residue designations do not always correspond directly with the linear numbering of the amino acid residues in SEQ ID sequences. The actual linear amino acid sequence may contain fewer or additional amino acids than in the strict Kabat numbering corresponding to a shortening of, or insertion into, a structural component, whether framework or complementarity determining region (CDR), of the basic variable domain structure. The correct Kabat numbering of residues may be determined for a given antibody by alignment of residues of homology in the sequence of the antibody with a "standard" Kabat numbered sequence. The CDRs of the heavy chain variable domain are located at residues 31-35B (H-CDR1), residues 50-65 (H-CDR2) and residues 95-102 (H-CDR3) according to the Kabat numbering system. The CDRs of the light chain variable domain are located at residues 24-34 (L-CDR1), residues 50-56 (L-CDR2) and residues 89-97 (L-CDR3) according to the Kabat numbering system. (http://www.bioinf.org.uk/abs/#cdrdef)

In the context of the invention, the amino acid residues of the antibody of the invention may be numbered according to the IMGT numbering system. The IMGT unique numbering has been defined to compare the variable domains whatever the antigen receptor, the chain type, or the species (Lefranc M.-P., "Unique database numbering system for immunogenetic analysis" Immunology Today, 18, 509 (1997) ; Lefranc M.-P., "The IMGT unique numbering for Immunoglobulins, T cell receptors and Ig-like domains" The Immunologist, 7, 132-136 (1999).; Lefranc, M.-P., Pommié, C., Ruiz, M., Giudicelli, V., Foulquier, E., Truong, L., Thouvenin-Contet, V. and Lefranc, G., "IMGT unique numbering for immunoglobulin and T cell receptor variable domains and Ig superfamily V-like domains" Dev. Comp. Immunol., 27, 55-77 (2003).). In the IMGT unique numbering, the conserved amino acids always have the same position, for instance cysteine 23, tryptophan 41, hydrophobic amino acid 89, cysteine 104, phenylalanine or tryptophan 118. The IMGT unique numbering provides a standardized delimitation of the framework regions (FR1-IMGT: positions 1 to 26, FR2-IMGT: 39 to 55, FR3-IMGT: 66 to 104 and FR4-IMGT: 118 to 128) and of the complementarity determining regions: CDR1-IMGT: 27 to 38, CDR2-IMGT: 56 to 65 and CDR3-IMGT: 105 to 117. If the CDR3-IMGT length is less than 13 amino acids, gaps are created from the top of the loop, in the following order 111, 112, 110, 113, 109, 114, etc. If the CDR3-IMGT length is more than 13 amino acids, additional positions are created between positions 111 and 112 at the top of the CDR3-IMGT loop in the following order 112.1,111.1, 112.2, 111.2, 112.3, 111.3, etc. (http://www.imgt.org/IMGTScientificChart/Nomenclature/IMGT-FRCDRdefinition.html).

As used herein, the term "antibody" denotes conventional antibodies and fragments thereof, as well as single domain antibodies and fragments thereof, in particular variable heavy chain of single domain antibodies, and chimeric, humanized, bispecific or multispecific antibodies.

As used herein, antibody or immunoglobulin also includes "single domain antibodies" which have been more recently described and which are antibodies whose complementary determining regions are part of a single domain polypeptide. Examples of single domain antibodies include heavy chain antibodies, antibodies naturally devoid of light chains, single domain antibodies derived from conventional four-chain antibodies, engineered single domain antibodies. Single domain antibodies may be derived from any species including, but not limited to mouse, human, camel, llama, goat, rabbit and bovine. Single domain antibodies may be naturally occurring single domain antibodies known as heavy chain antibody devoid of light chains. In particular, Camelidae species, for example camel, dromedary, llama, alpaca and guanaco, produce heavy chain antibodies naturally devoid of light chain. Camelid heavy chain antibodies also lack the CH1 domain.

The variable heavy chain of these single domain antibodies devoid of light chains are known in the art as "VHH" or "nanobody". Similar to conventional VH domains, VHHs contain four FRs and three CDRs. Nanobodies have advantages over conventional antibodies: they are about ten times smaller than IgG molecules, and as a consequence properly folded functional nanobodies can be produced by in vitro expression while achieving high yield. Furthermore, nanobodies are very stable, and resistant to the action of proteases. The properties and production of nanobodies have been reviewed by Harmsen and De Haard (2007) Appl. Microbiol. Biotechnol. 77:13-22.

The term "monoclonal antibody" or "mAb" as used herein refers to an antibody molecule of a single amino acid composition that is directed against a specific antigen, and is not to be construed as requiring production of the antibody by any particular method. A monoclonal antibody may be produced by a single clone of B cells or hybridoma, but may also be recombinant, *i.e.* produced by protein engineering.

"Fragments" of (conventional) antibodies comprise a portion of an intact antibody, in particular the antigen binding region or variable region of the intact antibody. Examples of antibody fragments include Fv, Fab, F(ab')₂, Fab', dsFv, (dsFv)₂, scFv, sc(Fv)₂, diabodies, bispecific and multispecific antibodies formed from antibody fragments. A fragment of a conventional antibody may also be a single domain antibody, such as a heavy chain antibody or VHH.

The term "Fab" denotes an antibody fragment having a molecular weight of about 50,000 Da and antigen binding activity, in which about a half of the N-terminal side of H chain and the entire L chain, among fragments obtained by treating IgG with a protease, papain, are bound together through a disulfide bond.

The term "F(ab')₂" refers to an antibody fragment having a molecular weight of about 100,000 Da and antigen binding activity, which is slightly larger than the Fab bound via a disulfide bond of the hinge region, among fragments obtained by treating IgG with a protease, pepsin.

A single chain Fv ("scFv") polypeptide is a covalently linked VH::VL heterodimer which is usually expressed from a gene fusion including VH and VL encoding genes linked by a peptide-encoding linker. The human scFv fragment of the invention includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)₂.
"dsFv" is a VH::VL heterodimer stabilized by a disulphide bond.
"(dsFv)₂" denotes two dsFv coupled by a peptide linker.

The term "bispecific antibody" or "BsAb" denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term "multispecific antibody" denotes an antibody which combines the antigen-binding sites of two or more antibodies within a single molecule.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites.

In a particular embodiment, the epitope-binding fragment is selected from the group consisting of Fv, Fab, F(ab')₂, Fab', dsFv, (dsFv)₂, scFv, sc(Fv)₂, diabodies and VHH.

A "chimeric antibody", as used herein, is an antibody in which the constant region, or a portion thereof, is altered, replaced, or exchanged, so that the variable region is linked to a constant region of a different species, or belonging to another antibody class or subclass. "Chimeric antibody" also refers to an antibody in which the variable region, or a portion thereof, is altered, replaced, or exchanged, so that the constant region is linked to a variable region of a different species, or belonging to another antibody class or subclass.

The term "humanized antibody" refers to an antibody which is initially wholly or partially of non-human origin and which has been modified to replace certain amino acids, in particular in the framework regions of the heavy and light chains, in order to avoid or minimize an immune response in humans. The constant domains of a humanized antibody are most of the time human CH and CL domains. In an embodiment, a humanized antibody has constant domains of human origin. As used herein, the term "humanized antibody" refers to a chimeric antibody which contain minimal sequence derived from non-human immunoglobulin, e.g. the CDRs.

The term "antibody" is used to encompass all these kinds of antibodies, fragments or combination thereof.

The goal of humanization is a reduction in the immunogenicity of a xenogenic antibody, such as a murine antibody, for introduction into a human, while maintaining the full antigen binding affinity and specificity of the antibody. Humanized antibodies, or antibodies adapted for non-rejection by other mammals, may be produced using several technologies such as resurfacing and CDR grafting. As used herein, the resurfacing technology uses a combination of molecular modeling, statistical analysis and mutagenesis to alter the non-CDR surfaces of antibody variable regions to resemble the surfaces of known antibodies of the target host.

Antibodies can be humanized using a variety of other techniques including CDR-grafting (EP0239400; WO91/09967; U.S. Patent Nos. 5,530,101 and 5,585,089), veneering or resurfacing (EP0592106; EP0519596; Padlan (1991) Molecular Immunology 28(4/5):489-498; Studnicka et al. (1994) Protein Engineering 7(6):805-814; Roguska et al. (1994) Proc. Natl. Acad. Sci U.S.A. 91:969-973), and chain shuffling (U.S. Patent No. 5,565,332). Human antibodies can be made by a variety of methods known in the art including phage display methods. See also U.S. Patent Nos. 4,444,887, 4,716,111, 5,545,806, and 5,814,318; and International patent application WO98/46645, WO98/50433, WO98/24893, WO98/16654, WO96/34096, WO96/33735, and WO91/10741.

As used herein, the term "specificity" refers to the ability of an antibody to detectably bind an epitope presented on an antigen, such as netrin-1, while having relatively little detectable reactivity with non-netrin-1 proteins or structures (such as other proteins presented on cancer cells, or on other cell types). Specificity can be relatively determined by binding or competitive binding assays, using, e.g., Biacore instruments, as described elsewhere herein. Specificity can be exhibited by, e.g., an about 10:1, about 20:1, about 50:1, about 100:1, 10.000:1 or greater ratio of affinity/avidity in binding to the specific antigen versus nonspecific binding to other irrelevant molecules (in this case the specific antigen is netrin-1).

The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Preferred methods for determining the affinity of mAbs can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983), which references are entirely incorporated herein by reference. One preferred and standard method well known in the art for determining the affinity of mAbs is the use of Biacore instruments.

### Administration of drugs, use, and method of use:

The pharmaceutical compositions can be administered to a patient at a suitable dose or at a therapeutically effective amount, *i.e.* for the anti-netrin-1 antibody at least 1 mg/kg body weight, e.g. about 1 mg/kg body weight to about 100 mg/kg body weight, in particular about 10 mg/kg body weight to about 60 mg/kg body weight of the patient in which inflammation is to be treated.

By the term "treating liver or hepatic inflammation" or "treatment of liver or hepatic inflammation" as used herein is meant in particular the reduction or suppression of hepatic inflammation. It may comprise the prevention of inflammation occurrence or its increase, in case of onset of inflammation.

In an aspect, this treatment allows reducing or suppressing the hepatic inflammation during all or part of the period during which the patient is treated with the antibody according to the invention.

In an aspect, the therapeutic effect of the anti-netrin-1 antibody on the hepatic inflammation may be monitored, or assessed in the patient. This can be made on liver biopsy or, more conveniently, on blood samples. For example, this is performed by measuring aminotransferase levels in the blood, and a therapeutic effect is obtained when the levels are below the level before treatment, and/or is ≤ 60 IU/ml, such as 5-40 IU/ml. In particular, the alanine aminotransferase (ALT, alanine transaminase) level is measured, and therapeutic effect is obtained when the serum level of ALT becomes below the upper limit of nomal (ULN), known from the clinician. Serum level decrease of ALT to ≤ 60IU/ml, such as 5-40 IU/ml, may be of significance. One may also use the hepatic activity index or score or any other available method. Thus, the method or use according to the invention may comprise treating the patient in need thereof with an antibody or composition of the invention, and monitoring or assessing dampening of liver inflammation using at least one of these means, especially the ALT measuring. This monitoring or assessment may be realized during the treatment period, or after the treatment period arrived to an end. It is also possible to perform more than one (e.g. 2 to 5, or more) monitoring or assessment during the treatment period and/or combining during and after treatment period.

The therapeutic effect may also be assessed through reversion of the induction of one or several transcripts, such as the chemokines *Ccl2*, *Ccl3, Ccl5, Ccl8,* and/or one or several actors related to the inflammation-associated interferon system, such as *Stat1, Oas1a, Oasl1, Ifi44*, and/or of the master regulator of inflammation cyclooxygenase *Ptgs2* and its product prostaglandin I2 *(Ptgir).*

The therapeutic effect may also be assessed through reversion of the transcripts encoding the chemoattractant *Cxcl10, Ccl2* and *Ccl5,* the canonical inflammatory cytokine *IL1B*, the macrophage inflammatory protein *Mip2* and the *Stat1* and *Gnb1* transducers.

Several of these methods may be applied. Thus, the method or use according to the invention may comprise treating the patient in need thereof with an antibody or composition of the invention, and monitoring or assessing dampening of liver inflammation by determining that there has been reversion of one or several of these transcripts. This monitoring or assessment may be realized during the treatment period, or after the treatment period arrived to an end. It is also possible to perform more than one (e.g. 2 to 5, or more) monitoring or assessment during the treatment period and/or combining during and after treatment period.

As an alternative or in addition to at least one of the above methods, the therapeutic effect may also be assessed through reversion of Ly6C⁺ intrahepatic macrophages counts, and/or through dampening of TLR2/6-driven hepatic inflammation triggered by the lipopeptide FSL-1, as well as of TLR3-driven inflammation triggered by HCV or double-stranded RNA. Thus, the method or use according to the invention may comprise treating the patient in need thereof with an antibody or composition of the invention, and monitoring or assessing dampening of liver inflammation by determining reversion of Ly6C⁺ intrahepatic macrophages counts, and/or dampening this TLR2/6-driven and/or TLR3-driven hepatic inflammation. This monitoring or assessment may be realized during the treatment period, or after the treatment period arrived to an end. It is also possible to perform more than one (e.g. 2 to 5, or more) monitoring or assessment during the treatment period and/or combining during and after treatment period.

According to the invention, the term "patient" or "patient in need thereof" is intended for a human or non-human mammal affected or likely to be affected with a liver inflammation or a liver inflammation associated with a disease such as : a liver infection (e.g. hepatitis B virus, hepatitis C virus); a metabolic disease; a drug induced liver injury (DILI) ; a chronic liver disease (CLD) whatever its etiology (e.g. viral infection, metabolic disease, alcohol related liver disease, autoimmune liver disease etc.); a toxic liver injury; an impaired liver function; cirrhosis; a genetic liver disease.

In an aspect, the treatment with the anti-netrin-1 antibody is combined with a treatment against the cause (etiology) of the disease as mentioned at the preceding paragraph. The combination may comprise the simultaneous, sequential or separate administration to the patient of the anti-netrin-1 antibody, and the active principle used to treat said disease.

By a "therapeutically effective amount" is meant a sufficient amount of the anti-netrin-1 antibody to treat said inflammation, at a reasonable benefit/risk ratio applicable to any medical treatment. It will be understood, however, that the total daily usage of the active agents will be decided by the attending physician within the scope of sound medical judgment. The specific therapeutically effective dose level for any particular patient will depend upon a variety of factors including the disorder being treated and the severity of the disorder; activity of the specific polypeptide or antibody employed; the specific composition employed, the age, body weight, general health, sex and diet of the patient; the time of administration, route of administration, and rate of excretion of the specific active agents employed; the duration of the treatment; drugs used in combination or coincidental with the specific active agents employed; and like factors well known in the medical arts.

### Pharmaceutical compositions:

The antibody is generally presented in a pharmaceutical composition with a pharmaceutically acceptable vehicle, carrier or excipient. The form of the pharmaceutical compositions including the antibody of the invention and the route of administration naturally depend upon the condition to be treated, the severity of the illness, the age, weight, and gender of the patient, etc.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The active agents of the invention can be formulated for a topical, oral, parenteral, intranasal, intravenous, intramuscular, subcutaneous or intraocular administration and the like. In a particular embodiment, the active agents of the invention are administered intravenously

In particular, the pharmaceutical compositions including the active agents of the invention may contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

To prepare pharmaceutical compositions, an effective amount of the active agents of the invention may be dissolved or dispersed in a pharmaceutically acceptable carrier or aqueous medium.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

The carrier can be a solvent or dispersion medium containing, for example, water, ethanol, polyol (for example, glycerol, propylene glycol, and liquid polyethylene glycol, and the like) and suitable mixtures thereof. The proper fluidity can be maintained, for example, by the use of a coating, such as lecithin, by the maintenance of the required particle size in the case of dispersion and by the use of surfactants, stabilizing agents, cryoprotectants or antioxidants. The prevention of the action of microorganisms can be brought about by antibacterial and antifungal agents. In many cases, it will be preferable to include isotonic agents, for example, sugars or sodium chloride.

Sterile injectable solutions are prepared by incorporating the active agents in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The invention will now be described using non-limiting examples referring to the Figures (or Fig.).

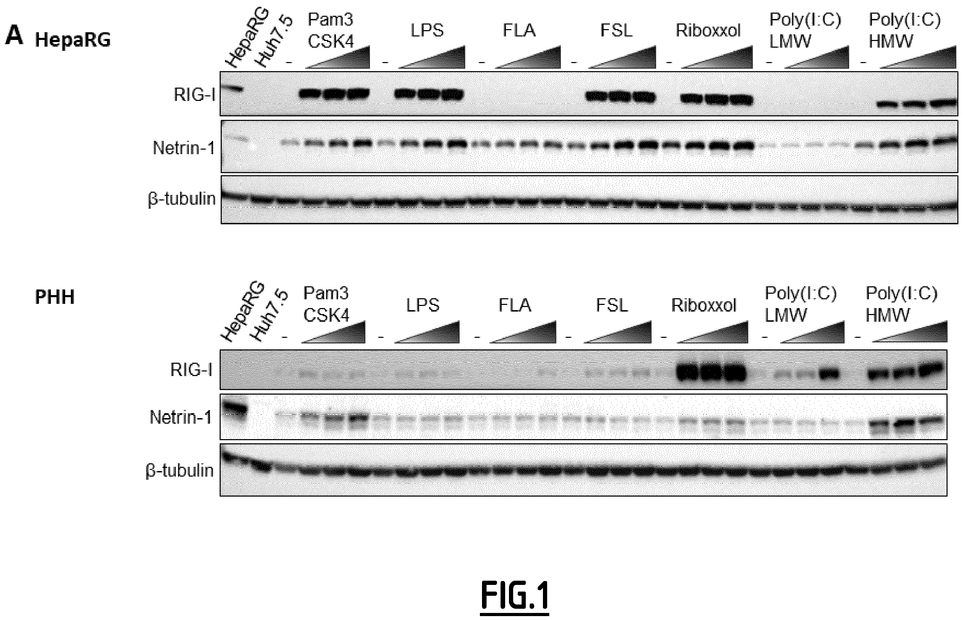

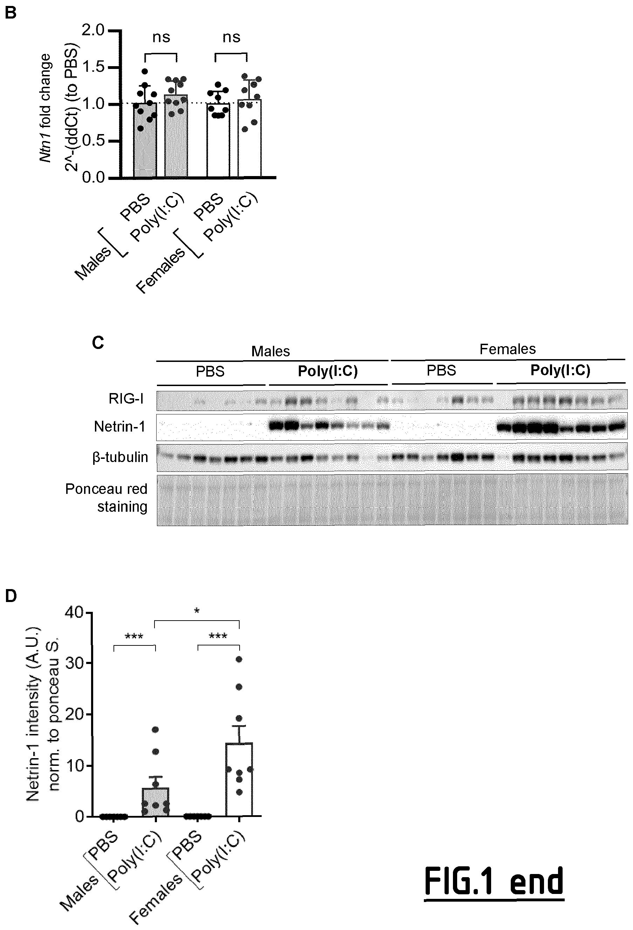

### Figure 1: Netrin-1 induction in vivo and in vitro.

**A.** Netrin-1 expression levels were evaluated by immunoblotting (n=3). **B.** Levels of *NTN1* mRNA in total RNA extracts processed from mouse livers were quantified by RT-qPCR. **C.** Same livers extracts were probed for immunoblotting. **D.** Densitometric quantification of netrin-1 on mice blots. Data are representative of a total of 40 mice (10 females, 10 males, +/- poly(I:C), Mann-Whitney test (n.s).

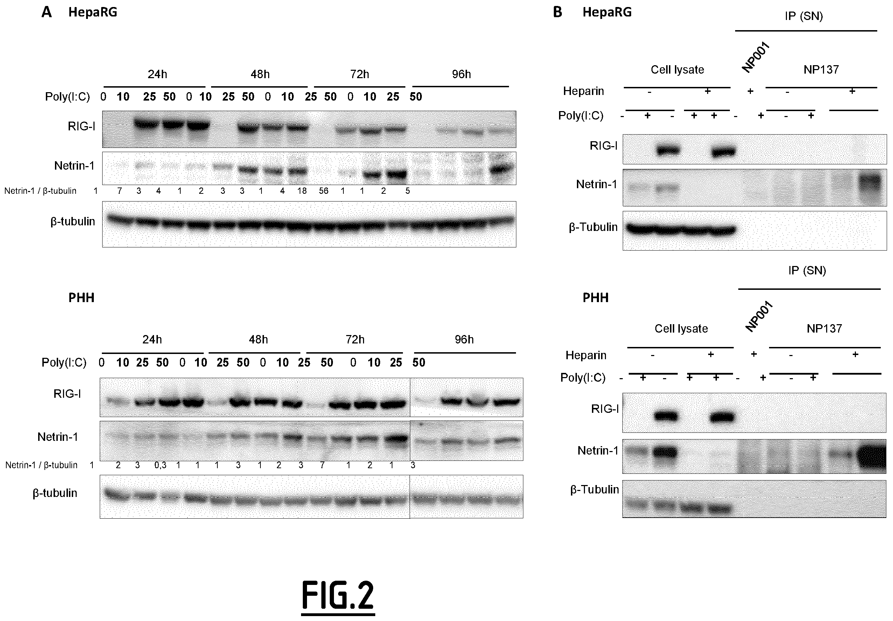

### Figure 2: Cell-associated and soluble netrin-1 levels are upregulated by poly(I:C) in HepaRG cells and in PHH.

**A.** Netrin-1 expression levels were evaluated by immunoblotting. **B.** Netrin-1 expression levels were evaluated by immunoblotting in total cell lysates or after immunoprecipitation in supernatants, with or without heparin pretreatment. NP001: isotype control. NP137: anti-netrin-1 Ig. RIG-I was used as a poly(I:C) activity control (n = 3, HepaRG; and n = 3 distinct PHH batches in both panels).

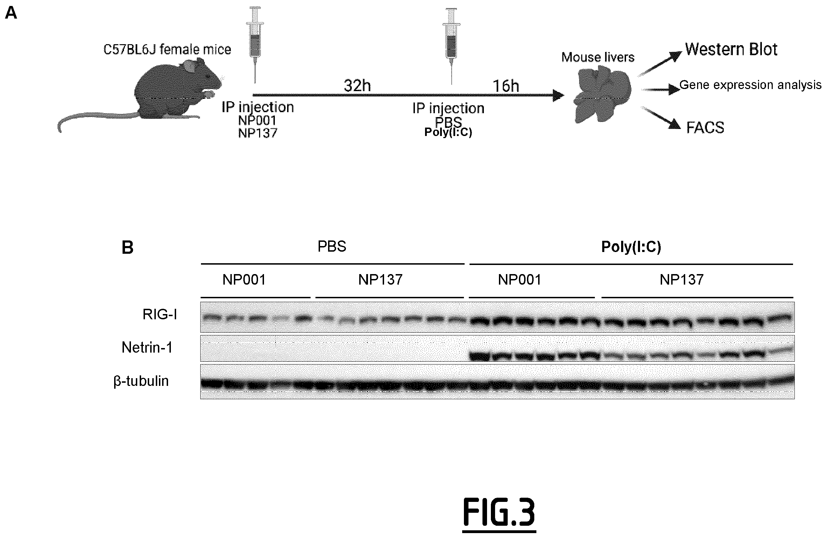

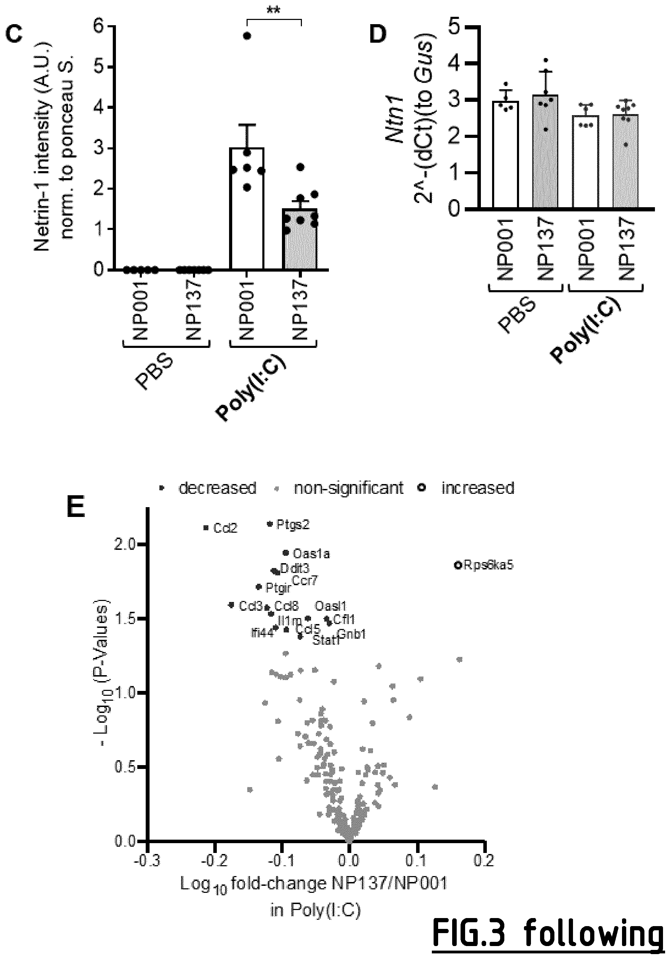

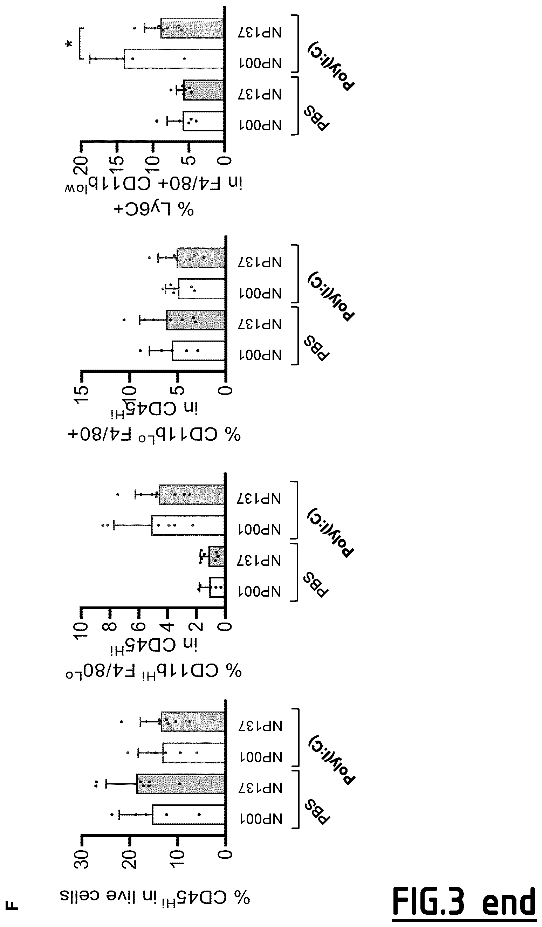

### Figure 3. Netrin-1 capture by a clinically used antibody dampens hepatic inflammation in mice.

**A.** Workflow created with Biorender.com. **B.** Hepatic netrin-1 levels in mice were measured by western blotting. **C.** Quantification of signals. **D**. *Ntn1* was quantified by RT-qPCR. **E.** Transcripts conditioned by netrin-1 levels. F. Percentage of positive live (DsRed) cells against CD45, CD11b, F4/80, Ly6C markers are indicated (n = 5-8 mice per group). Mann-Whitney test. *p < 0.05, **p<0.01, ***p<0.001.

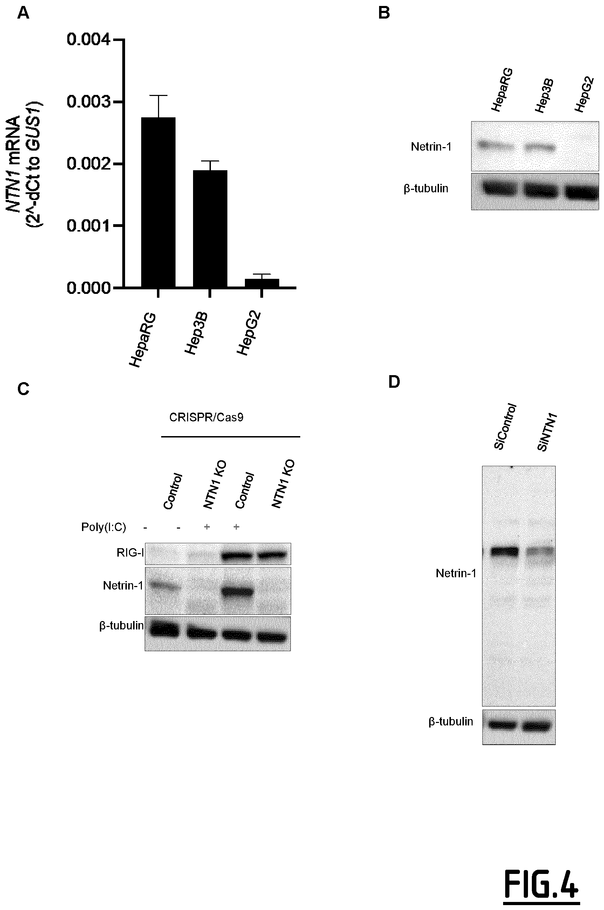

**Figure 4****. In-house assessment of anti-netrin-1 antibody reliability. A-B.** A panel of hepatocyte-like cell lysates derived from NTN1 mRNA negative or positive cells was probed by RT-qPCR and netrin-1 Western blotting, respectively, using the Abcam reference Ab126729. **C-D.** CRISPR/Cas-9-engineered netrin-1 KO HepaRG cells and NTN1 siRNAtreated HEK293T cells, respectively, were subjected to anti-netrin-1 immunoblotting using the same antibody.

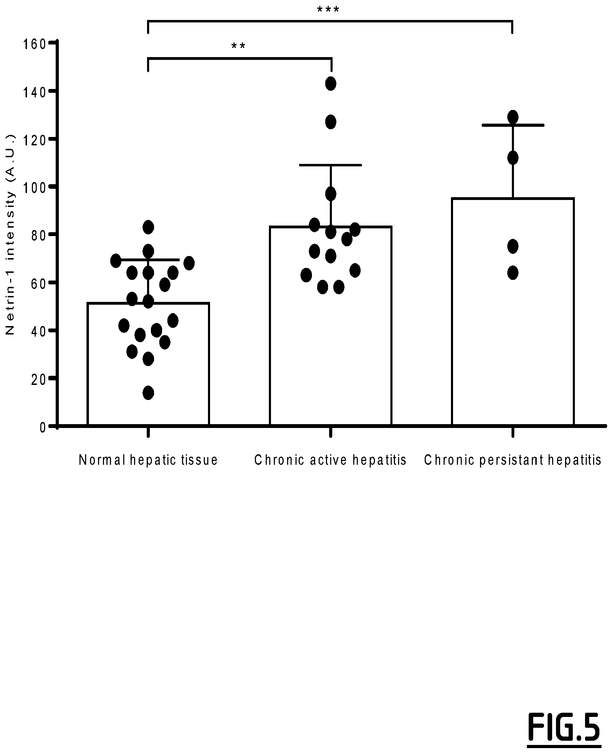

**Figure 5****. Netrin-1 correlation with inflammation in clinical samples.** Netrin-1 levels were quantified by IHC in 34 CLD patients samples spotted onto commercial TMAs.

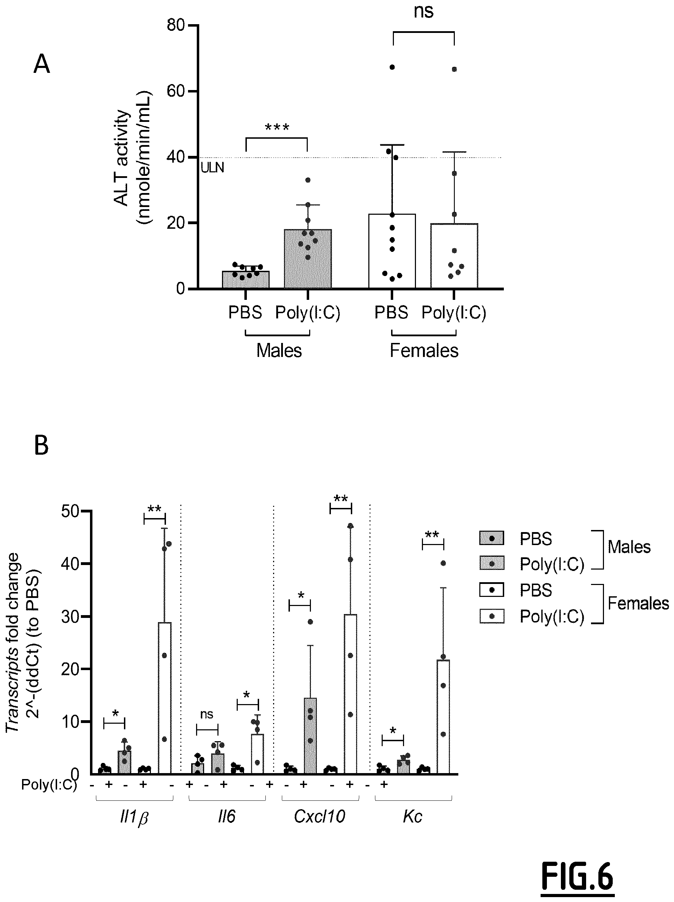

**Figure 6****. Poly(I:C) activity assessment in mice. A.** ALT activity was monitored in the serum of the same mice challenged with poly(I:C). n = 10 females and 10 males, +/poly(I:C), corresponding to 40 animals. * p < 0.05, ** p < 0.01, Mann-Whitney test after Shapiro-Wilk test. ULN, upper limit of normal values. **B.** C57BL/6J mice were challenged with poly(I:C), their liver harvested, and total RNA extraction processed for RT-qPCR on inflammation-related transcripts. Corresponding canonical markers of inflammation activation (IL1B (β), IL6, CXCL10, KC (ortholog of hIL8)) were monitored (for each one order on the abscissa is PBS Males, Poly(I:C) Males, PBS Females, Poly(I:C) Females.

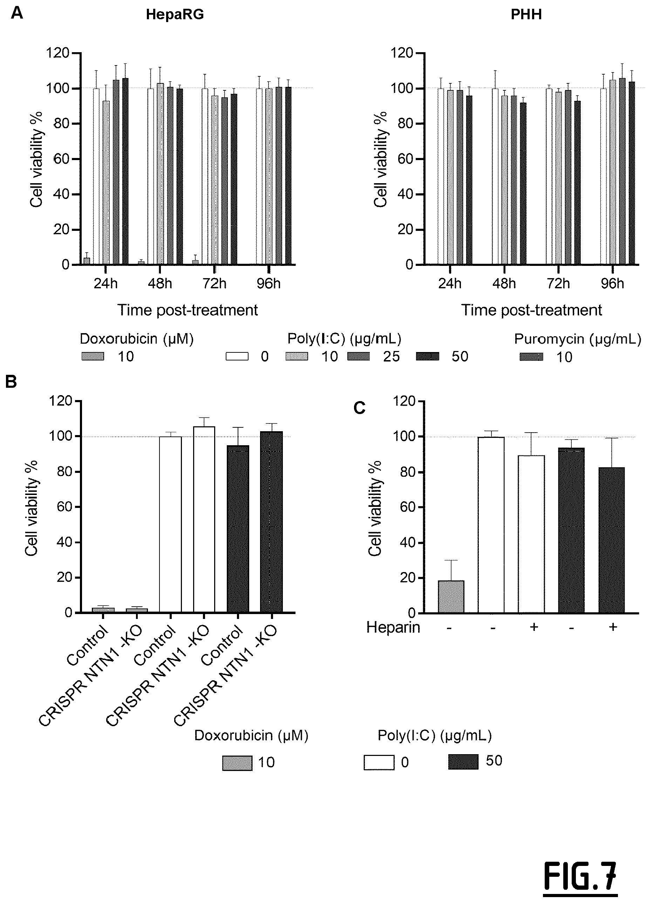

**Figure 7****. Netrin-1 induction is not related to cell death in HepaRG cells and PHH.** Viability was quantified by the Neutral Red assay using doxorubicin and puromycin as death inducers controls, respectively. **A.** n = 3 (HepaRG); n = 3 distinct PHH batches (in this order on he abscissa: Doxo, Poly(I:C) 0, 10, 25, 50 µg/mL, puromycin) **B.** n = 3 (Netrin-1-KO and control CRISPR/Cas-9 HepaRG). Mann-Whitney test (n.s.). **C.** Cells were treated with heparin overnight prior to harvest (HepaRG, n = 3, n.s).

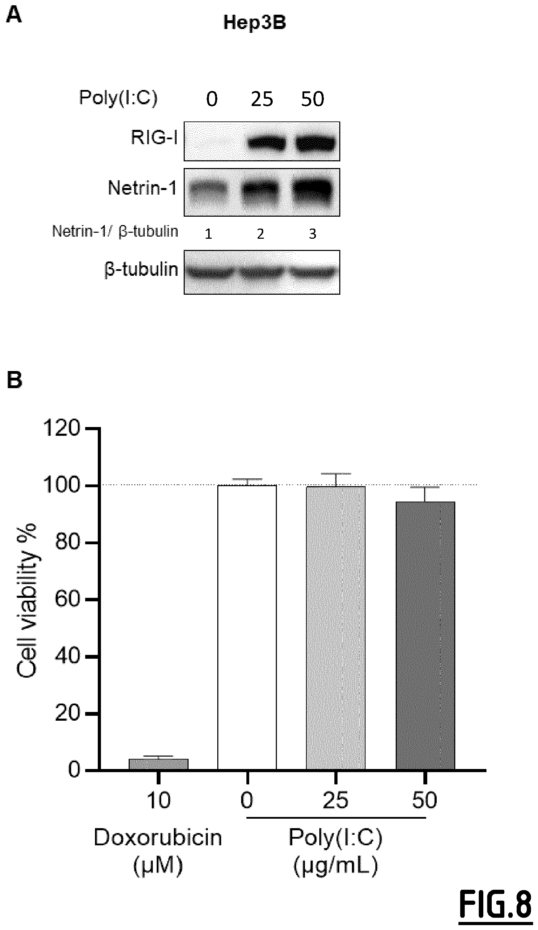

**Figure 8****. Netrin-1 induction is not related to cell death in Hep3B cells. A.** Netrin-1 expression levels were evaluated by immunoblotting in Hep3B cells (n=3 independent experiments). RIG-I was used as a poly(I:C) activity control. **B.** Viability was quantified by the Neutral Red assay using doxorubicin as a death control (n = 3). Mann-Whitney test (n.s.).

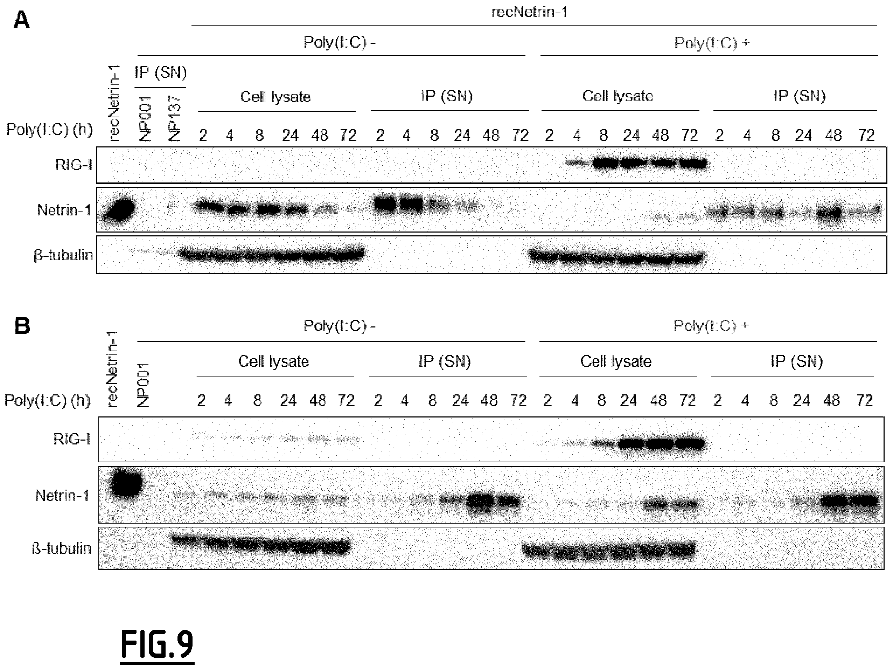

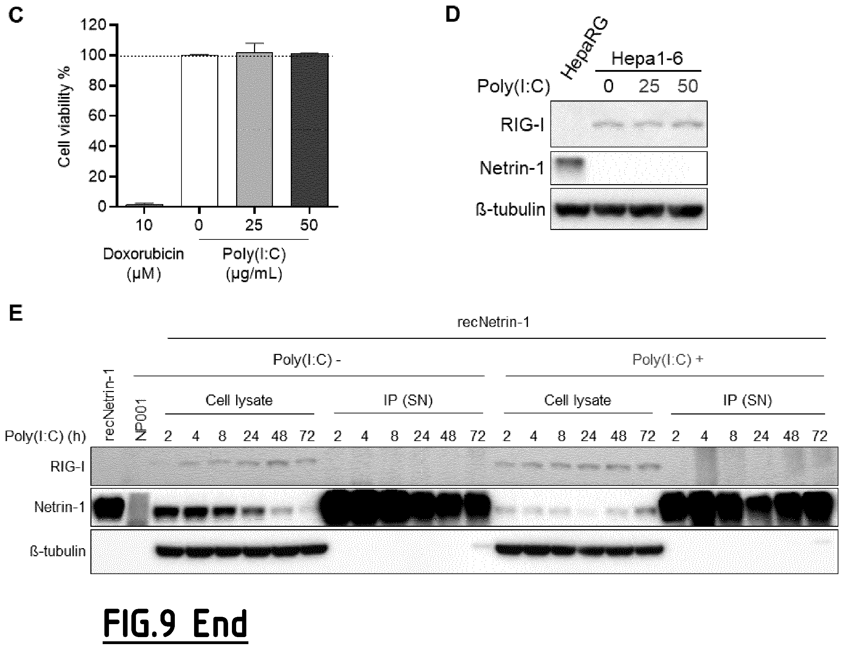

**Figure 9****. Inflammation mobilizes netrin-1 from its cell-associated pool.** Netrin-1 KO CRISPR/Cas9 HepaRG cells **(A),** PHH **(B)** and Hepa1-6 cells **(C-E),** were pre-treated with recombinant netrin-1 (previously in-house validated for activity) for 4 h, and subsequently incubated with poly(I:C). for the indicated times. Cell viability and netrin-1 null status were determined for Hep1-6 cells **(C-D).** Cells were then submitted to total lysates-and IP-based immunoblotting for RIG-I as a poly(I:C) activity control and netrin-1, respectively. Tubulin probing of the blotted membrane was performed to ascertain purity of extracellular IP material. Representative of three independent experiments.

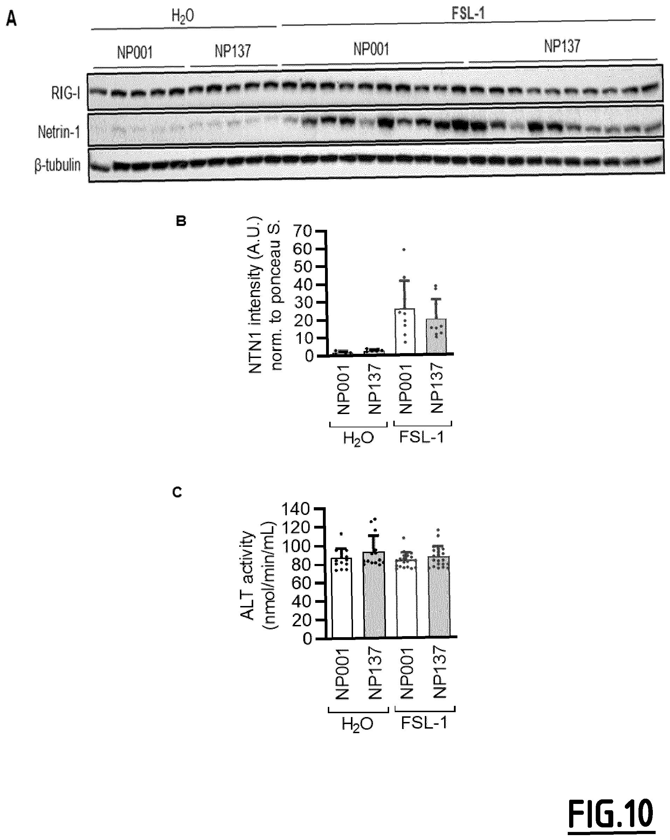

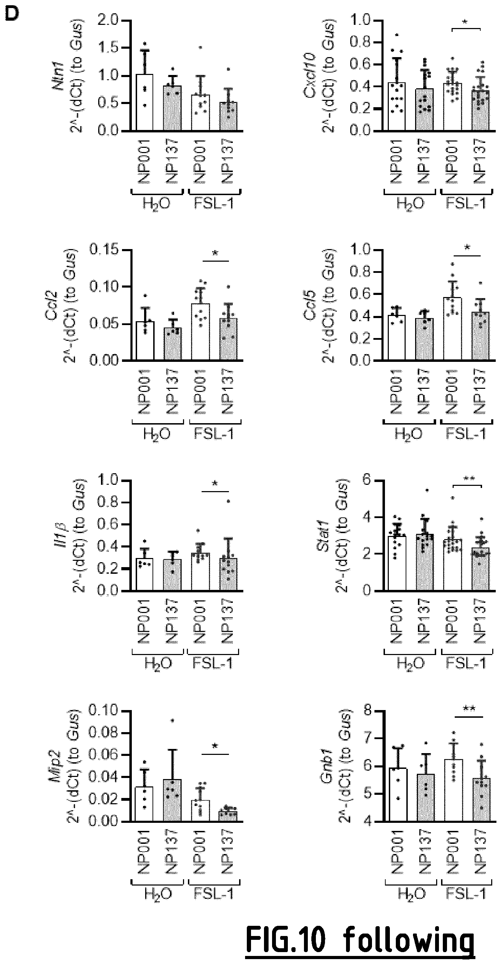

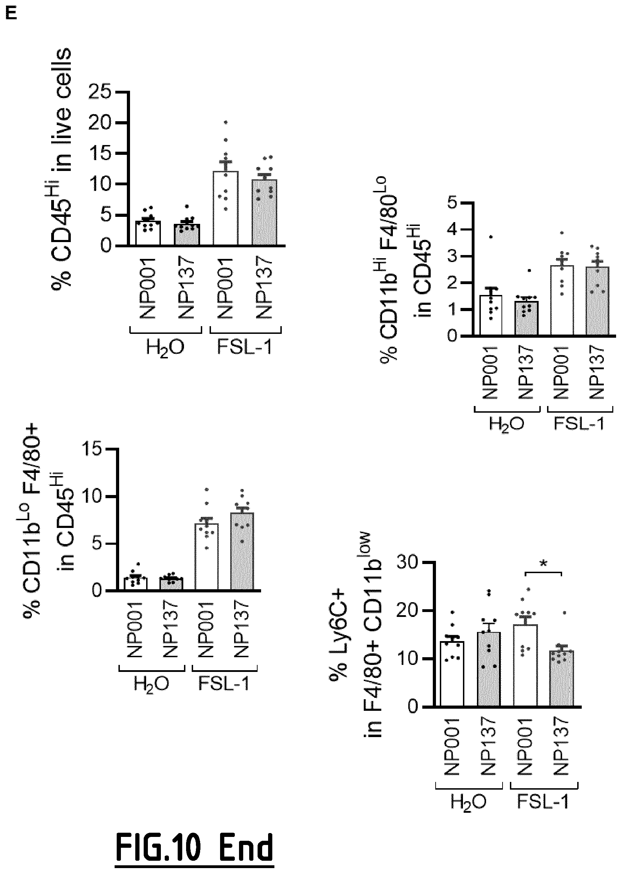

**Figure 10****. Netrin-1 capture by a clinically used antibody dampens hepatic inflammation in mice. A.** Hepatic netrin-1 levels in mice pre-treated with control NP001 or anti-netrin-1 NP137 antibody and subjected to PBS or poly(I:C) treatment were measured by western blotting. **B.** Quantification of netrin-1 signals by densitometry. **C.** ALT activity quantification. **D.** *Ntn1* regulation and identification of 7 inflammation-related transcripts that are conditioned by netrin-1 levels. **E.** Percentage of positive live (DsRed) cells against CD45, CD11b, F4/80, Ly6C markers are indicated in the presence or absence of poly(I:C) and netrin-1 inactivation (n= 10 mice per group). Mann-Whitney test. *p < 0.05, **p<0.01, ***p<0.001.

### Results

### 1. Correlation between inflammation and netrin-1 in the clinic

In order to evaluate the clinical likelihood of an interplay between netrin-1 and inflammation in the liver, we first considered a potential association between netrin-1 and inflammation in clinical samples, performing immunohistochemistry (after extensive anti-netrin-1 antibody validation, **shown in** **Fig. 4****)** on a series of 34 commercial TMA samples **(****Figure 5****)**. Such data indicate that netrin-1 protein levels are correlated with liver inflammation in TMA patients.

### 2. Inflammation fosters netrin-1 protein upregulation in vitro and in mice.

In order to probe the robustness of these correlative clinical data, we investigated the potential impact of experimentally-induced inflammation, using a panel of TLR ligands, namely Pam3-CSK4 as a TLR1/2 agonist, lipopolysaccharide (LPS) as a TLR4 agonist, FLA as a TLR5 agonist, FSL as a TLR2/6 agonist, Riboxxol (molecularly defined TLR3 agonist), and finally low (LMW) or high molecular weight (HMW) poly(I:C) as TLR3, RIG-I and MDA-5 agonists. Experiments were carried out on HepaRG cells (1) and in primary human hepatocytes (PHH). RIG-I was also probed as a control. Pam3CSK4, LPS, FSL, Riboxxol, and HMW poly(I:C) induced netrin-1, suggesting its relevance in the hepatic environment. Inducibility was confirmed in PHH for Pam3CSK4, LPS, Riboxxol and HMW poly(I:C). **(****Fig. 1A****).** Chronic viral liver infections represent one of the major aetiologies of CLDs, and, though still controversial for HBV, HBV and HCV have been reported to be direct inducers of dsRNA-based responses (2, 3), which in turn unleash an inflammatory response in hepatocytes. TLR3 signalling may be activated by a variety of ligands, derived from HCV infection or other viral motifs (4), commensal bacteria (5) or hepatotoxicity (6). We hereafter focused on the dsRNA analogue poly(I:C) of HMW, as a viral mimetic and TLR3 agonist, and robust netrin-1 inducer.

We initially assayed inflammation-related netrin-1 induction in the liver *in vivo.* To achieve this, C57BL/6J male and female mice were challenged with HMW-poly(I:C), prior to harvesting their liver. Although poly(I:C) injection did not increase ALT levels in mouse serum in females and marginally in males **(****Fig. 6A****),** several other markers of inflammation *(CXCL10, IL1B*, *IL6, KC)* were induced in both sexes. Interestingly, the inducibility of inflammation was higher in females **(****Fig. 6B****).** Though *NTN1* mRNA induction was not observed within total RNA populations, a Western blot and IHC approach uncovered netrin-1 induction, and to a greater extent in female mice (**Fig. 1 B****-E**). Such data suggest a causal link between inflammation, inflammation intensity, and netrin-1 induction. By IHC, netrin-1 staining yielded a homogenous, and therefore most likely hepatocytic as well, staining pattern, similar to previous data obtained in human liver (7).

To gain further insight into the mechanisms of netrin-1 production, we conducted a set of *in vitro* experiments using HepaRG cells (1) as well as PHH, where poly(I:C) led to netrin-1 protein induction in a dose- and time-dependent manner (56- and 7-fold increase in HepaRG and PHH after 72 h, respectively). The RIG-I inducible marker was monitored to verify poly(I:C) activity **(****Fig. 2A****).** As a laminin-related protein, netrin-1 is secreted and may accumulate at the extracellular surface of the plasma membrane. We thus tested netrin-1 signals by immunoprecipitation after heparin-based release of plasma membrane-bound netrin-1. Data indicate that the previously observed netrin-1 upregulation concerns the plasma membrane-bound netrin-1 pool in both cell types **(****Fig. 2B****).**

### 3. Netrin-1 is mobilized towards its soluble form by poly(I:C)

As published in several studies, tissular inflammation may be altered by netrin-1 (8-11), suggesting its mobilization in interstitial fluids. Also, poly(I:C) exerts anti-HBV activity through inflammation-associated processes (12). To test whether netrin-1 may act as an auto/paracrine factor with diffusible consequences, we monitored the behaviour of exogenously added in-house validated (7) recombinant netrin-1, on netrin-1 KO Crispr HepaRG cells or netrin-1 null Hepa1-6 mouse cells upon poly(I:C) treatment. Using netrin-1 immunoprecipitation and Western blot, we observed that poly(I:C) mobilized netrin-1 within 2 hours from its cell-associated pool towards supernatants. Same data were obtained with endogenous netrin-1, using PHH **(****Fig. 9****),** hence arguing in favour of its role at the microenvironmental level in the hepatocytic environment.

Importantly, netrin-1 upregulation by inflammation was unrelated to cell death protection (see below).

### 4. Netrin-1 induction is unrelated to cell death.

We then tested if netrin-1 upregulation was independent of its well-documented activity as a protector against cell death. We monitored cell viability upon poly(I:C) and heparin treatment, using the neutral red assay in wild-type HepaRG, PHH, and CRISPR/Cas9 netrin-1 KO HepaRG cells and could not evidence cell death upon treatment **(****Fig. 7****).** Such results were also found in a hepatocarcinoma cell line Hep3B **(****Fig. 8****).** Data indicate that during inflammation, netrin-1 induction is not related to its ability to counteract cell injury and is likely involved in cell death-unrelated processes.

### 5. A phase 2 trial-validated antibody enabling netrin-1 capture unravels its contribution to hepatic inflammation in mice challenged with TLR2/3/6-interacting microbial motifs.

Considering that the diffusible status of netrin-1 corroborated previously known tissular effects of netrin-1 (8-11), we treated C57BL/6J mice with the clinically used and well tolerated anti-netrin-1 antibody NP137 (NCT02977195) (13) or its NP001 isotypic control 32h before challenging them with poly(I:C) for 16h and liver harvest **(****Fig. 3A****).** Treatment with the former dampened poly(I:C)-induced netrin-1 levels **(****Fig. 3B-C****)** in addition of its netrin-1 capture activity (13). Such events occurred again independently of *NTN1* RNA upregulation in all instances **(****Fig. 3D****).** Gene expression analysis was conducted on a panel of 248 inflammation-related cytokines. Poly(I:C)-triggered upregulation of 81 inflammation-related transcripts, see the following **Table, Gene expression raw intensities data:**

| **Transcript** | **Mean (s.d) PBS** | **Mean (s.d) Poly(I:C)** | **Fold-change vs PBS** | ***P*** |
|---|---|---|---|---|
| *Arg1* | 36358 (3683) | 45668 (3168) | 1.26 | 1.5E-03 |
| *Bcl2l1* | 683 (83) | 990 (136) | 1.45 | 1.8E-03 |
| *C1ra* | 2659 (456) | 3144 (239) | 1.18 | 4.9E-02 |
| C2 | 2568 (257) | 5313 (521) | 2.07 | 2.0E-06 |
| *C3ar1* | 276 (68) | 421 (88) | 1.52 | 1.6E-02 |
| C6 | 915 (189) | 574 (165) | 0.63 | 1.1E-02 |
| *C8b* | 4857 (965) | 6755 (1408) | 1.39 | 3.1E-02 |
| *Ccl19* | 79 (15) | 160 (34) | 2.03 | 7.6E-04 |
| *Ccl2** | 120 (21) | 791 (258) | 6.60 | 2.8E-04 |
| *Ccl24* | 162 (40) | 68 (16) | 0.42 | 4.3E-04 |
| *Ccl3** | 20 (0) | 69 (25) | 3.46 | 1.7E-03 |
| *Ccl4* | 20 (0) | 45 (11) | 2.23 | 6.2E-04 |
| *Ccl5** | 81 (17) | 1944 (351) | 24.06 | 9.1E-07 |
| *Cc*/*7* | 42 (11) | 88 (15) | 2.10 | 3.3E-04 |
| *Ccl8** | 30 (10) | 74 (15) | 2.44 | 3.3E-04 |
| *Ccr2* | 85 (26) | 142 (27) | 1.67 | 6.3E-03 |
| *Ccr7** | 26 (7) | 38 (4) | 1.47 | 6.7E-03 |
| *Cd163* | 332 (108) | 122 (41) | 0.37 | 1.6E-03 |
| *Cd40* | 27 (5) | 48 (13) | 1.77 | 6.7E-03 |
| *Cd55* | 861 (248) | 1209 (147) | 1.40 | 1.8E-02 |
| *Cd86* | 173 (36) | 249 (61) | 1.44 | 3.8E-02 |
| *Cdc42* | 8256 (449) | 10268 (707) | 1.24 | 3.9E-04 |
| *Cfb* | 20896 (2959) | 27046 (3028) | 1.29 | 8.0E-03 |
| *Cfl1** | 3740 (378) | 5500 (411) | 1.47 | 4.4E-05 |
| *Chi3l3* | 20 (0) | 62 (22) | 3.11 | 1.9E-03 |
| *Csf1* | 354 (49) | 471 (55) | 1.33 | 4.9E-03 |
| *Cxcl10* | 143 (42) | 619 (179) | 4.33 | 2.7E-04 |
| *Cxcl9* | 833 (182) | 47992 (11288) | 57.59 | 7.0E-06 |
| *Daxx* | 153 (19) | 227 (24) | 1.48 | 3.7E-04 |
| *Ddit3** | 173 (44) | 972 (124) | 5.61 | 2.6E-07 |
| *Fos* | 286 (142) | 126 (32) | 0.44 | 2.4E-02 |
| *Fxyd2* | 20 (0) | 24 (4) | 1.20 | 3.3E-02 |
| *Gnb1** | 2549 (166) | 3245 (210) | 1.27 | 2.1E-04 |
| *Grb2* | 784 (43) | 874 (42) | 1.11 | 7.0E-03 |
| *Hsh2d* | 20 (0) | 22 (2) | 1.11 | 4.3E-02 |
| *Ifi27l2a* | 798 (167) | 2630 (410) | 3.30 | 7.0E-06 |
| *Ifi44** | 428 (129) | 3619 (809) | 8.45 | 1.2E-05 |
| *Ifit1* | 402 (97) | 1263 (400) | 3.14 | 1.2E-03 |
| *Ifit2* | 314 (60) | 1247 (232) | 3.97 | 1.1E-05 |
| *Ifit3* | 1398 (221) | 5608 (1203) | 4.01 | 3.2E-05 |
| *Iigp1* | 8651 (1519) | 15546 (2943) | 1.80 | 1.1E-03 |
| *Il11* | 40 (8) | 60 (12) | 1.50 | 1.2E-02 |
| *Il15* | 38 (9) | 56 (11) | 1.45 | 2.0E-02 |
| *Il1a* | 174 (32) | 208 (12) | 1.19 | 3.7E-02 |
| *Il1rn** | 33 (5) | 41 (4) | 1.26 | 1.3E-02 |
| *Il7* | 39 (7) | 59 (10) | 1.50 | 5.7E-03 |
| *Irf7* | 694 (210) | 6089 (853) | 8.78 | 2.5E-07 |
| *Itgb2* | 222 (56) | 488 (154) | 73.24 | 5.5E-03 |
| *Limk1* | 72 (9) | 88 (8) | 4.966 | 1.2E-02 |
| *Ltb4r2* | 20 (0) | 24 (3) | 1.528 | 4.0E-02 |
| *Ly96* | 346 (38) | 395 (35) | 21.8 | 4.9E-02 |
| *Map2k1* | 1508 (58) | 1766 (90) | 46.71 | 3.7E-04 |
| *Mapk14* | 1277 (68) | 1437 (118) | 59.8 | 2.5E-02 |
| *Mbl2* | 20906 (4913) | 36840 (7174) | 3797 | 2.3E-03 |
| *Mmp9* | 27 (4) | 38 (5) | 3.05 | 6.6E-03 |
| *Mrc1* | 1589 (422) | 1053 (96) | 176 | 1.4E-02 |
| *Mx1* | 107 (32) | 240 (43) | 23.14 | 2.8E-04 |
| *Mx2* | 239 (88) | 415 (47) | 41.45 | 2.1E-03 |
| *Myd88* | 285 (14) | 355 (41) | 19.21 | 5.7E-03 |
| *Nfatc3* | 415 (27) | 494 (29) | 17.03 | 1.3E-03 |
| *Nfe2l2* | 2017 (240) | 2875 (327) | 176.7 | 9.0E-04 |
| *Oas1a** | 331 (61) | 2560 (345) | 157.5 | 1.9E-07 |
| *Oas2* | 106 (42) | 182 (50) | 28.15 | 2.4E-02 |
| *Oasl1** | 602 (175) | 1587 (216) | 120.4 | 1.8E-05 |
| *Ptger3* | 24 (4) | 20 (0) | 1.625 | 4.4E-02 |
| *Ptgir** | 20 (0) | 40 (8) | 3.387 | 2.7E-04 |
| *Ptgs2** | 20 (0) | 32 (5) | 2.094 | 3.5E-04 |
| *Ptk2* | 538 (50) | 624 (47) | 29.37 | 1.7E-02 |
| *Rapgef2* | 809 (38) | 951 (69) | 34.88 | 2.8E-03 |
| *Relb* | 119 (25) | 169 (32) | 17.58 | 1.9E-02 |
| *Rps6ka5*** | 59 (15) | 36 (10) | 7.622 | 1.5E-02 |
| *Smad7* | 200 (28) | 275 (57) | 28.15 | 2.6E-02 |
| *Stat1** | 1465 (228) | 3685 (594) | 283.5 | 2.6E-05 |
| *Tlr1* | 51 (6) | 155 (57) | 25.83 | 3.0E-03 |
| *Tlr3* | 56 (18) | 84 (18) | 10.81 | 3.0E-02 |
| *Tlr4* | 93 (19) | 127 (27) | 14.42 | 4.2E-02 |
| *Tlr6* | 34 (11) | 50 (11) | 6.55 | 3.4E-02 |
| *Tlr9* | 37 (8) | 98 (20) | 9.618 | 1.3E-04 |
| *Tnf* | 20 (0) | 33 (6) | 2.628 | 1.0E-03 |
| *Tnfaip3* | 142 (33) | 183 (19) | 15.84 | 2.8E-02 |
| *Tollip* | 1303 (124) | 1082 (138) | 79.67 | 2.1E-02 |
| *Tradd* | 113 (19) | 138 (15) | 10.2 | 3.5E-02 |
| *Traf2* | 126 (24) | 162 (19) | 12.84 | 2.0E-02 |

| | | | | |
|---|---|---|---|---|
| * Down-regulated by NP137 ** Up-regulated by NP137 | | | | |

Our experiment highlighted a causal relationship between netrin-1 inactivation by NP137 and reversion of the induction of 15 transcripts, of which several encode chemokines (*Ccl2*, -1.63-fold; *Ccl3* -1.51-fold; *Ccl5* -1.64-fold; *Ccl8,* -1.33-fold). Four actors related to the inflammation-associated interferon system (*Stat1, Oas1a, Oasl1, Ifi44*) were also significantly reversed, albeit more weakly, together with the master regulator of inflammation cyclooxygenase *Ptgs2* and its product prostaglandin I2 (*Ptgir*)*.* Consistently, the only NP137-induced transcript out of the 248 analysed targets encodes an anti-inflammatory kinase, *Rps6ka5* (+1.44-fold, **Fig. 3E**). In order to further document this phenotype that implicates several chemotactic factors, we performed a flow cytometry analysis to monitor immune cell population regulation. Monocyte-derived macrophages (CD45⁺ CD11b⁺ F4/80^{low}) remained stable. Total Kupffer cells (CD45⁺ CD11b^{low} F4/80^{hi}) counts were not regulated. However, the NP137 antibody specifically reversed Ly6C⁺ intrahepatic macrophages counts in inflammatory conditions **(****Fig. 3F****),** a population that is activated by CCL2, CCL3 and CCL5 in four distinct models of chronic liver disease (14-16).

Confirmatory data were obtained in the context of lipopeptide FSL-1-driven inflammation. Because of their bacterial antigen-like status and behavior, other compounds than poly(I:C) have also relevance in liver inflammation, in particular when related to pathological translocation of bacterial antigens towards the liver in NASH, alcoholic liver disease and associated cirrhosis. Using the same experimental outline as for poly(I:C), we therefore tested the ability of NP137 to dampen TLR2/6-driven hepatic inflammation triggered by the lipopeptide FSL-1. As was the case before, frank induction of netrin-1, in the absence of any cytolysis, could be observed. Amongst the previously tested candidates, NP137 significantly reversed the transcripts encoding the chemoattractant *Cxcl10, Ccl2* and *Ccl5* as was the case with poly(I:C), the canonical inflammatory cytokine *IL1B*, the macrophage inflammatory protein *Mip2* and the *Stat1* and *Gnb1* transducers. Importantly, again, the NP137 antibody specifically reversed Ly6C⁺ intrahepatic macrophage counts in inflammatory conditions **(****Fig. 10****).** No increase of any proinflammatory factor could be observed. Hence, such data identify netrin-1 as an amplifier of liver inflammation induced by two unrelated microbial classes of triggers (dsRNA and lipopeptides), in accordance with its role in other organs (8-10). Thus, the NP137 antibody is a potential tool to dampen hepatic inflammation.

### Material and Methods:

### Cell culture and siRNA/plasmids transfection

Cells were grown in a 5% CO₂ humidified atmosphere at 37°C. All reagents pertaining to HepaRG cell culture were purchased from ThermoFischer Scientific (Les Ulis, France) unless otherwise indicated. HepaRG cells were seeded at a density of 4.10⁴/cm² in William's E medium containing 5 mg/mL insulin (Sigma, Saint-Quentin, France, 5.10⁻⁵ mol/L hydrocortisone hemisuccinate (Merck, Fontenay, France), glutamax 1X, penicillin 100 IU/mL, streptomycin 100 mg/mL, and 10% FBS (Hyclone, Illkirch, France). Hep3B cells were obtained from S. Rebouissou after STR characterisation and cultured in DMEM (ThermoFischer, Les Ulis, France) (17). Medium was renewed twice a week. Primary human hepatocytes (PHHs) were isolated from surgical liver resections, after informed consent of patients (IRB agreements #DC-2008-99 and DC-2008-101) as previously described (18) and cultured in complete William's medium supplemented with 1.8% DMSO (Sigma, St Quentin, France). All PHH-related data were obtained from at least three distinct patients. For TLR3-specific or pan-TLR stimulation, cells were treated with Pam3-CSK4, LPS, Riboxxol (0, 1.25, 2.5 and 5µg/ml), with FLA or FSL (0.675, 1.25, 2.5µg/ml) or with LMW or HMW poly(I:C) (0, 10, 25, 50 µg/ml, all from InvivoGen, Toulouse, France) four days after seeding (i.e. after having reached confluency (HepaRG) or optimal polarisation (PHH)) for the indicated time points and/or concentrations.

### Western blotting

Immunoblotting was performed using 40 µg of RIPA (50 mM Tris HCI (pH 8.0), 150 mM NaCl, 1% NP-40, 0.5% sodium deoxycholate, 0.1% SDS, 10 mM sodium fluoride, 50 mM orthovanadate, 1× protease inhibitor cocktail (Roche))-processed cell lysates, then resolved on 8% or 10% SDS-PAGE, blotted onto nitrocellulose membranes (Amersham Biosciences, Saclay, France), blocked using 5% low fat dried milk in TBS Tween 0.1% for 1 h at RT and probed overnight at 4°C with corresponding antibodies. After three washes in PBS-Tween 0.1%, membranes were incubated for 1 h at room temperature with secondary antibodies coupled to HRP (1/5,000, Sigma-Aldrich, St-Quentin, France) prior to chemiluminescence-based revelation using the Clarity Western ECL substrate (Bio-Rad, Versailles, France). The Western blot specificity of the netrin-1 antibody was extensively verified in-house prior to conducting experiments **(****Fig. 4****).** Densitometry was performed using the Image Lab software.

### Netrin-1 Crispr cell lines

A netrin KO CRISPR cell line and its ad hoc control cell line were generated. The Zhang lab protocol (19) was used. Briefly, guide sequences were inserted into lentiCRISPR v2 (Addgene plasmid #52961) and lentiCRISPR v2-Blast (Addgene plasmid #83480). DNAs (Gag-Pol + Env + lentiCRISPRv2 or lentiCRISPRv2 Blast) were transfected into 2 × 10⁶ HEK293T cells (ATCC CRL-1573) seeded the day before in 10-cm plates using calcium phosphate (20). The medium (8 mL/plate) was replaced 16 h after transfection. Virus-containing supernatants were harvested 24 h later, filtered through 0.45-µm pore-sized membranes, and stored at -80°C until further transduction. For selection of the resulting polyclonal cell lines, cells were plated at a density of 4.10⁴/cm² in a 35 mm Petri dish. The next day, lentiviral transduction was done using 250 µL of each lentivirus supernatant for 0.5 million cells, for two days. Two days later, cells were then put under selective pressure for the expression of both lentiviral cassettes using puromycin and blasticidin (Invivogen, gradually increasing concentrations from 4 and 8 µg/mL to 10 and 20 µg/mL within two weeks, comprising two weekly trypsinization procedures). Cell lysates were then generated for netrin-1 KO monitoring by Western blot using the anti-netrin-1 reference Ab126729 (Abcam) prior to liquid nitrogen storage.

### Flow cytometry analysis

Pieces of liver were minced and digested in RPMI-1640 supplemented with mouse tumor dissociation enzymes (Miltenyi Biotec, Bergisch Gladbach, Germany) for 40 min at 37°C. Digested samples were filtered through a 70 µm nylon mesh to obtain uniform single-cell suspensions. Cells were washed with complete RPMI medium containing 10% decomplemented foetal bovine serum (FBS), 1% NEAA, 1% Sodium Pyruvate and 1% Penicillin-Streptomycin (Gibco^{™}) and resuspended in 500 µL of Flow Cytometry Staining Buffer (FCSB) (eBioscience^{™}). 100 µL of cell suspension was used for staining in U-shaped 96-well plates. After PBS 1X washing, cells were stained with LIVE/DEAD^{™} Fixable Red Dead Cell Stain Kit for 30 min protected from light at room temperature. Fc receptors were blocked with anti-CD16/CD32 (TruStain FcX^{™}; Biolegend) and cells were stained for cell surface antigens:

### Antibodies.

| **Antigen** | **Ig Species** | **Cat. Number & supplier** | **Antibody registry (RRID) #** |
|---|---|---|---|
| CD45 | Rat IgG2b, κ | APC-Cy7 Biolegend | AB_312981, clone: 30-F11 |
| CD11b | Rat IgG2b, κ | 10705, Stem Cells Technologies | AB_215609 |
| Ly6C | Rat IgG2c, κ | BLE128017 | ND |

For intracellular staining, cells were fixed and permeabilized using the Foxp3 / Transcription Factor Staining Buffer Set (eBioscience^{™}). Events were acquired on a BD-LSRII flow cytometer (BD Biosciences, Le Pont-De-Claix, France), collected with the BD FACSDiva 6.3.1 software and analysed using the Flowlogic software.

### Netrin-1 immunoprecipitation

Culture media, serum-free or not, complemented or not with heparin overnight (200 µg/mL, Sigma-Aldrich, Saint-Quentin, France), were applied to the cells overnight, 60 h after poly(I:C) treatment. Five x or 1x lysis buffer (20 mM Tris pH 7.5, 150 mM NaCl, 1 mM EDTA, 1 mM EGTA, 0.1% Triton X-100, Protease inhibitor cocktail 1×) were used for supernatant / combined samples or total lysates immunoprecipitations, respectively. Two µg of anti-netrin-1 NP137 antibody (13) / 5 mL of medium or mg of protein extracted from total lysates were incubated overnight on a rotating wheel at 4°C. Twenty µL of SureBeads^{™} Protein A Magnetic Beads (Biorad, Marnes, France) was added for 2 µg of antibody, for 1 h on a rotating wheel at 4°C. Beads were then washed three times with lysis buffer 1× and eluted in 20 µL of 1× Laemmli buffer for 5 min at 95°C, then extracted with acid phenol (Sigma, Saint-Quentin, France) as described earlier (21).

### Netrin-1 release assay

For netrin-1 release assays, CRISPR control (HepaRG-Ctrl-KO) and netrin-1 KO cell lines were seeded as depicted above, allowed to reach confluency for 4 days, and sequentially treated with recombinant soluble netrin-1 (12.5 ng/mL, R&D systems) followed by 50 µg/mL poly(I:C) for the indicated times, before supernatant harvesting and its clarification (1,000 *g,* 5 min, 4°C). Monolayers and supernatants were washed with ice-cold PBS and processed for netrin-1 immunoprecipitation and western blotting.

### Immunohistochemistry

For histological examination, tissue samples were fixed in 10% buffered formalin and embedded in paraffin and prepared according to conventional procedures. Immunohistochemistry was performed on an automated immunostainer (Ventana Discovery XT, Roche, Meylan, France) using the Discovery HRP Kit according to the manufacturer's instructions. Sections were incubated with an anti-netrin-1 antibody (1/500; anti-netrin-1 Abcam ref Ab126729). Staining was visualized with DAB solution with 3,3'-diaminobenzidine as a chromogenic substrate. Finally, the sections were counterstained with Gill's haematoxylin.

### Cell viability and ALT assays

The Neutral Red (NR) assay was conducted as described by Repetto (22). Briefly, the NR stock solution (40 mg NR dye in 10 mL PBS) was diluted in culture medium to a final concentration of 4 mg/mL and then centrifuged at 600 g for 10 min to remove any precipitated dye crystals. Cells were then incubated with 100 µL of NR medium for 1 h. NR medium was removed and the cells washed with PBS. Plates were incubated for 10 min under shaking with 150 µL/well of NR destain solution (50% ethanol 96%, 49% deionized water, 1% glacial acetic acid). OD at 540 nm was measured. Mouse sera ALT activity values were calculated using the colorimetric function of the ALT quantification kit (MAK-052, Sigma-Aldrich).

### References

1. Parent R, Marion MJ, Furio L, Trepo C, Petit MA. Origin and characterization of a human bipotent liver progenitor cell line. Gastroenterology 2004;126:1147-1156.
2. Saito T, et al. Innate immunity induced by composition-dependent RIG-I recognition of hepatitis C virus RNA. Nature 2008;454:523-527.
3. Sato S, et al. The RNA sensor RIG-I dually functions as an innate sensor and direct antiviral factor for hepatitis B virus. Immunity 2015;42:123-132.
4. Yu P, et al. Nucleic acid-sensing Toll-like receptors are essential for the control of endogenous retrovirus viremia and ERV-induced tumors. Immunity 2012;37:867-879.
5. Kawashima T, et al. Double-stranded RNA of intestinal commensal but not pathogenic bacteria triggers production of protective interferon-beta. Immunity 2013;38:1187-1197.
6. Cavassani KA, et al. Toll like receptor 3 plays a critical role in the progression and severity of acetaminophen-induced hepatotoxicity. PLoS One 2013;8:e65899.
7. Plissonnier ML, et al. Epidermal Growth Factor Receptor-Dependent Mutual Amplification between Netrin-1 and the Hepatitis C Virus. PLoS Biol 2016;14:e1002421.
8. Hadi T, et al. Macrophage-derived netrin-1 promotes abdominal aortic aneurysm formation by activating MMP3 in vascular smooth muscle cells. Nat Commun 2018;9:5022.
9. Mediero A, et al. Netrin-1 and its receptor Unc5b are novel targets for the treatment of inflammatory arthritis. FASEB J 2016;30:3835-3844.
10. van Gils JM, et al. The neuroimmune guidance cue netrin-1 promotes atherosclerosis by inhibiting the emigration of macrophages from plaques. Nat Immunol 2012;13:136-143.
11. Schlegel M, et al. The neuroimmune guidance cue netrin-1 controls resolution programs and promotes liver regeneration. Hepatology 2015.
12. Wu J, Huang S, et al. Poly(I:C) treatment leads to interferon-dependent clearance of hepatitis B virus in a hydrodynamic injection mouse model. J Virol 2014;88:10421-10431.
13. Grandin M, et al. Structural Decoding of the Netrin-1/UNC5 Interaction and its Therapeutical Implications in Cancers. Cancer Cell 2016;29:173-185.
14. Ambade A, et al. Pharmacological Inhibition of CCR2/5 Signaling Prevents and Reverses Alcohol-Induced Liver Damage, Steatosis, and Inflammation in Mice. Hepatology 2019;69:1105-1121.
15. Baeck C, et al. Pharmacological inhibition of the chemokine C-C motif chemokine ligand 2 (monocyte chemoattractant protein 1) accelerates liver fibrosis regression by suppressing Ly-6C(+) macrophage infiltration in mice. Hepatology 2014;59:1060-1072.
16. Baeck C, et al. Pharmacological inhibition of the chemokine CCL2 (MCP-1) diminishes liver macrophage infiltration and steatohepatitis in chronic hepatic injury. Gut 2012;61:416-426.
17. Caruso S, et al. Analysis of Liver Cancer Cell Lines Identifies Agents With Likely Efficacy Against Hepatocellular Carcinoma and Markers of Response. Gastroenterology 2019;157:760-776.
18. Lecluyse EL, Alexandre E. Isolation and culture of primary hepatocytes from resected human liver tissue. Methods Mol Biol 2010;640:57-82.
19. Sanjana NE, Shalem O, Zhang F. Improved vectors and genome-wide libraries for CRISPR screening. Nat Methods 2014;11:783-784.
20. Bassot A, et al. Regulation of Mitochondria-Associated Membranes (MAMs) by NO/sGC/PKG Participates in the Control of Hepatic Insulin Response. Cells 2019;8.
21. Parent R, Kolippakkam D, Booth G, Beretta L. Mammalian target of rapamycin activation impairs hepatocytic differentiation and targets genes moderating lipid homeostasis and hepatocellular growth. Cancer research 2007;67:4337-4345.
22. Repetto G, del Peso A, Zurita JL. Neutral red uptake assay for the estimation of cell viability/cytotoxicity. Nat Protoc 2008;3:1125-1131.
23. Grandin M, et al. Structural Decoding of the Netrin-1/UNC5 Interaction and its Therapeutical Implications in Cancers. Cancer Cell 2016;29:173-185.

## Claims

1. An anti-netrin-1 antibody or an anti-netrin-1-binding fragment thereof for use in treating liver inflammation.

2. The antibody or fragment thereof for the use of claim 1, wherein the antibody specifically bind to a polypeptide of sequence SEQ ID NO: 33.

3. The antibody or fragment thereof for the use of claim 1 or 2, wherein the antibody or fragment thereof comprises
a variable domain VH comprising:
- a H-CDR1 having a sequence set forth as SEQ ID NO: 5;
- a H-CDR2 having a sequence set forth as SEQ ID NO: 6;
- a H-CDR3 having a sequence set forth as SEQ ID NO: 7;
a variable domain VL comprising:
- a L-CDR1 having a sequence set forth as SEQ ID NO: 8;
- a L-CDR2 having a sequence YAS;
- a L-CDR3 having a sequence set forth as SEQ ID NO: 9;
or
a variable domain VH comprising:
- a H-CDR1 having a sequence set forth as SEQ ID NO: 28;
- a H-CDR2 having a sequence set forth as SEQ ID NO: 29;
- a H-CDR3 having a sequence set forth as SEQ ID NO: 30;
a variable domain VL comprising:
- a L-CDR1 having a sequence set forth as SEQ ID NO: 31;
- a L-CDR2 having a sequence set forth as SEQ ID NO: 32;
- a L-CDR3 having a sequence set forth as SEQ ID NO: 9.

4. The antibody or fragment thereof for the use of any one of claims 1 to 3, wherein the antibody or fragment thereof comprises a pair of VH and VL sequences selected from the following pairs: SEQ ID NO: 27 and 19, SEQ ID NO: 20 and 14, SEQ ID NO: 21 and 15, SEQ ID NO: 22 and 16, SEQ ID NO: 23 and 17, SEQ ID NO: 24 and 17, SEQ ID NO: 25 and 16, SEQ ID NO: 26 and 17, SEQ ID NO: 22 and 17, SEQ ID NO: 25 and 18, SEQ ID NO: 21 and 16, and preferably the antibody or fragment thereof comprises a pair of VH and VL sequences SEQ ID NO: 22 and 16.

5. The antibody for the use according to any one of claims 1 to 4, comprising a Human IgG1 Constant heavy chain (CH) and/or a Human IgG1 Constant light chain (CL), in particular a human kappa constant domain.

6. The antibody for the use according to any one of claims 1 to 5, comprising a VH of sequence of SEQ ID NO: 22 and a VL of sequence SEQ ID NO: 16.

7. The antibody for the use according to claim 6, further comprising a human IgG1 CH Genbank AEL33691.1 modified R97K, and a human IgG1 CL Kappa Genbank CAC20459.1.

8. A pharmaceutical composition comprising an antibody according to any one of claims 1 to 7, for use in treating liver inflammation.
